# EUROPEAN PATENT APPLICATION

(11) **EP 2 500 049 A1**
(43) Date of publication of application: **19.09.2012**
(21) Application number: 12169942.5
(22) Date of filing: 09.05.2008
(51) Int. Cl.: A61M 5/00

(54) **Wireless monitor for a personal medical device system**

(30) Priority: 01.06.2007 US 757153
(62) Divisional of application: 08795846.8
(71) Applicant: Medtronic MiniMed, Inc., Northridge, CA 91325-1219 (US)
(72) Inventor: Rankers, Ulrich, Livermore, CA 94550 (US); Enegren, Bradley J., Moorpark, CA 93021 (US); Gottlieb, Rebecca K., Culver City, CA 90232 (US); Han, Jino, Studio City, CA 91604 (US); Holtzclaw, Kris R., Santa Clarita, CA 91350 (US); Hong, Peter I., Valencia, CA 91354 (US); Istoc, Emilian, Winnetka, CA 91306 (US); Mastrototaro, John, J., Los Angeles, CA 90024 (US); Moberg, Sheldon, B., Thousand Oaks, CA 91362 (US); Talbot, Cary, Santa Clarita, CA 91350 (US); Williams, Gary, Gardena, CA 90247 (US); Halpern, Arieh, S., Beverly Hills, CA 90210 (US)
(74) Representative: Ruschke, Hans Edvard

(57) **Abstract**

A monitor device (200) for a medical device system (100) has a display element, a display controller /driver coupled to the display element and configured to generate a screen for rendering on the display element. The system further has processing architecture configured to analyze sensor data obtained by the monitor device, the sensor data indicating empirical measurements of a physiological characteristic of a monitored patient, and estimate future measurements of the physiological characteristic based upon the sensor data: The screen comprises a predictive graph (734) of the physiological characteristic that graphically indicates the empirical measurements and the future measurements. In an embodiment the predictive graph (734) comprises indicia of a target zone (740) for the physiological characteristic, the processing architecture being configured to predict whether the future measurements will leave the target zone (740) within a period of time, and to obtain an estimated time (746) corresponding to when the future measurements will leave the target zone (740); and wherein the screen comprises an indicator of the estimated time.

## Description

### TECHNICAL FIELD

The subject matter described herein relates generally to infusion systems that deliver fluids into a patient's body. More particularly, an embodiment of the subject matter described herein relates to a device that wirelessly monitors patient and status information generated by one or more devices within an infusion system.

### BACKGROUND

Diabetics are usually required to modify and monitor their daily lifestyle to keep their body in balance, in particular, their blood glucose (BG) levels. Individuals with Type 1 diabetes and some individuals with Type 2 diabetes use insulin to control their BG levels. To do so, diabetics routinely keep strict schedules, including ingesting timely nutritious meals, partaking in exercise, monitoring glucose levels daily, and adjusting and administering insulin dosages accordingly.

The prior art includes a number of insulin pump systems that are designed to deliver accurate and measured doses of insulin via infusion sets (an infusion set delivers the insulin through a small diameter tube that terminates at a cannula inserted under the patient's skin). In lieu of a syringe, the patient can simply activate the insulin pump to administer an insulin bolus as needed, for example, in response to the patient's current glucose level. A patient can measure his glucose level using a glucose measurement device, such as a test strip meter, a continuous glucose measurement system, or the like. Glucose measurement devices use various methods to measure the glucose level of a patient, such as a sample of the patient's blood, a sensor in contact with a bodily fluid, an optical sensor, an enzymatic sensor, or a fluorescent sensor. When the measurement device has generated a glucose measurement, the value is displayed on the measurement device. A continuous glucose monitoring system can monitor the patient's glucose level in real time.

Insulin pumps and continuous glucose monitoring devices may also be configured to communicate with remote control devices, monitoring or display devices, BG meters, and other devices associated with such an infusion system. Individual devices within conventional infusion systems may be configured to support a limited amount of wired or wireless data communication to support the operation of the infusion system. For example, a continuous glucose monitoring sensor may include a wireless transmitter that communicates with a glucose monitor device or an insulin pump within the infusion system. Moreover, an insulin pump device itself may include a display and monitoring functions for pump-related and/or patient-related data and alarms.

### BRIEF SUMMARY

An embodiment of a monitor device as described here is suitable for use with a personal medical device system, such as an insulin infusion system having an insulin pump. The monitor device is configured as a "bedside" or "tableside" monitor having a relatively large and easy-to-read display screen. The monitor device supports wireless data communication with a compatible insulin pump, which is implemented as a personal patient-worn device. The insulin pump transmits pump data, physiological patient data, alarm signals, and/or control signals to the monitor device, which processes the received data in an appropriate manner. In certain embodiments, the monitor device receives physiological patient data (such as glucose data) directly from a physiological sensor transmitter, and the monitor device processes the received physiological patient data in an appropriate manner. The monitor device includes a number of features and functions that enhance its operation and user interfaces, and make it easy to use.

The above and other aspects may be carried out by an embodiment of a monitor device for a fluid infusion system that includes a medical device having an exhaustible operating quantity. The monitor device includes: a display element; a display controller/driver coupled to the display element and configured to generate a screen for rendering on the display element; and a data communication module configured to receive current status data of the medical device, the current status data indicating a remaining measurement for the exhaustible operating quantity. The screen generated by the monitor device includes a status icon that graphically indicates the remaining measurement.

The above and other features may be carried out by an embodiment of a method of operating a monitor device for a fluid infusion system that includes a medical device having an exhaustible operating quantity. The method involves: receiving current status data of the medical device, the current status data indicating a remaining measurement for the exhaustible operating quantity; generating a status element that graphically indicates the remaining measurement relative to time; and displaying the status element at the monitor device.

The above and other aspects may be carried out by an embodiment of a monitor device for a fluid infusion system. The monitor device includes: a display element; a display controller/driver coupled to the display element and configured to generate a screen for rendering on the display element; and a processing architecture. The processing architecture is configured to analyze sensor data obtained by the monitor device, the sensor data indicating empirical measurements of a physiological characteristic of a monitored patient, and to estimate future measurements of the physiological characteristic based upon the sensor data. The screen generated by the monitor device includes a predictive graph of the physiological characteristic that graphically indicates the future measurements.

The above and other features may be carried out by an embodiment of a method of operating a monitor device for a fluid infusion system. The method involves: receiving sensor data that indicates empirical measurements of a physiological characteristic of a monitored patient; estimating future measurements of the physiological characteristic based upon the sensor data; generating a predictive graph of the physiological characteristic, where the predictive graph graphically indicates the future measurements; and displaying the predictive graph at the monitor device.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### BRIEF DESCRIPTION OF THE DRAWINGS

A more complete understanding of the subject matter may be derived by referring to the detailed description and claims when considered in conjunction with the following figures, wherein like reference numbers refer to similar elements throughout the figures.

FIG. 1 is a schematic representation of an embodiment of a fluid infusion system;

FIG. 2 is a perspective front view of an embodiment of a monitor device suitable for use in a fluid infusion system such as that shown in FIG. 1;

FIG. 3 is a perspective rear view of the monitor shown in FIG. 2;

FIG. 4 is a schematic representation of an embodiment of a monitor;

FIG. 5 is a schematic representation of processing logic and/or processing modules that may be implemented in an embodiment of a monitor;

FIG. 6 is a schematic representation of data types and/or information that may be stored and processed by an embodiment of a monitor;

FIG. 7 is a flow chart that illustrates an embodiment of a power up process for a monitor;

FIG. 8 is a front view of the monitor shown in FIG. 2, with a monitor screen displayed;

FIG. 9 is a front view of the monitor shown in FIG. 2, with another monitor screen displayed;

FIG. 10 is a front view of the monitor shown in FIG. 2, with a main menu screen displayed;

FIG. 11 is a front view of the monitor shown in FIG. 2, with an alarm history screen displayed;

FIG. 12 depicts a monitor alarm screen that may be generated by an embodiment of a monitor;

FIG. 13 depicts a pump alarm screen that may be generated by an embodiment of a monitor;

FIG. 14 depicts another pump alarm screen that may be generated by an embodiment of a monitor;

FIG. 15 depicts a high glucose alarm screen that may be generated by an embodiment of a monitor;

FIG. 16 depicts a low glucose alarm screen that may be generated by an embodiment of a monitor;

FIG. 17 depicts a sensor alarm screen that may be generated by an embodiment of a monitor;

FIG. 18 depicts a predictive glucose graph that may be generated by an embodiment of a monitor;

FIG. 19 is a flow chart that illustrates an embodiment of a status icon display process for a monitor; and

FIG. 20 is a flow chart that illustrates an embodiment of a predictive graph display process for a monitor.

### DETAILED DESCRIPTION

The following detailed description is merely illustrative in nature and is not intended to limit the embodiments of the invention or the application and uses of such embodiments. Furthermore, there is no intention to be bound by any expressed or implied theory presented in the preceding technical field, background, brief summary or the following detailed description.

Techniques and technologies may be described herein in terms of functional and/or logical block components and various processing steps. It should be appreciated that such block components may be realized by any number of hardware, software, and/or firmware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments may be practiced in conjunction with any number of data transmission protocols and that the system described herein is merely one suitable example.

For the sake of brevity, conventional techniques related to infusion system operation, insulin pump and/or infusion set operation, blood glucose sensing and monitoring, signal processing, data transmission, signaling, network control, and other functional aspects of the systems (and the individual operating components of the systems) may not be described in detail here. Examples of infusion pumps and/or communication options may be of the type described in, but not limited to, United States patent numbers: 4,562,751; 4,685,903; 5,080,653; 5,505,709; 5,097,122; 6,554,798; 6,558,320; 6,558,351; 6,641,533; 6,659,980; 6,752,787; 6,817,990; and 6,932,584, which are herein incorporated by reference. Examples of glucose sensing and/or monitoring devices maybe be of the type described in, but not limited to, United States patent numbers: 6,484,045; 6,809,653; 6,892,085; and 6,895,263, which are herein incorporated by reference. Furthermore, the connecting lines shown in the various figures contained here are intended to represent example functional relationships and/or physical couplings between the various elements. It should be noted that many alternative or additional functional relationships or physical connections may be present in an embodiment of the described subject matter.

The following description refers to elements or nodes or features being "connected" or "coupled" together. As used herein, unless expressly stated otherwise, "connected" means that one element/node/feature is directly joined to (or directly communicates with) another element/node/feature, and not necessarily mechanically. Likewise, unless expressly stated otherwise, "coupled" means that one element/node/feature is directly or indirectly joined to (or directly or indirectly communicates with) another element/node/feature, and not necessarily mechanically.

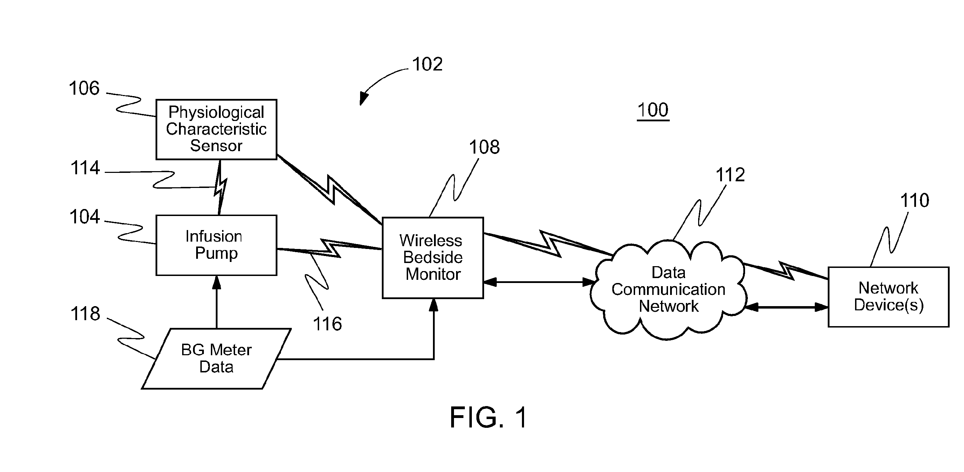

FIG. 1 is a schematic representation of an embodiment of a fluid infusion system 100. In this example, system 100 is an insulin infusion system that controls the infusion of insulin into the body of a user. Certain aspects of system 100, however, may also be utilized in the context of other medical device systems. Briefly, system 100 includes a local or personal infusion system 102 having one or more local devices that communicate (unidirectional or bidirectional) within local infusion system 102. For this simplified embodiment, local infusion system 102 includes an infusion pump 104, at least one physiological characteristic sensor 106, and at least one monitor 108. In certain embodiments of system 100, monitor 108 is suitably configured to communicate with one or more network devices 110. As used here, network devices 110 are "external" to local infusion system 102 because they need not utilize the local data communication protocols and techniques employed within local infusion system 102, and because they need not be in close physical proximity to the local devices within local infusion system 102. The manner in which monitor 108 communicates with a given network device 110 may vary depending upon the particular configuration of system 100, the specific characteristics of monitor 108, and the characteristics of that network device 110. For example, network communications may be routed using one or more data communication networks 112, which may employ wireless and/or wired data transport links.

For the illustrated embodiment, physiological characteristic sensor 106 and infusion pump 104 communicate with each other via at least one wireless link 114, while infusion pump 104 and monitor 108 communicate with each other via at least one wireless link 116. Alternatively (or additionally), physiological characteristic sensor 106 may communicate with infusion pump 104 via a wired link, physiological characteristic sensor 106 may communicate with monitor 108 via a wireless or wired link, and/or infusion pump 104 may communicate with monitor 108 via a wired link. In the preferred embodiment, infusion pump 104 is configured to wirelessly communicate with monitor 108. Accordingly, monitor 108 may be referred to herein as a wireless monitor 108. Wireless link 116 enables the patient (wearing or carrying infusion pump 104) to move freely relative to monitor 108, which may be designed to be placed on a nightstand, table, or windowsill, mounted to a wall, or the like. In other words, monitor 108 is designed for operation as a stationary unit without portability and mobility in mind. However, in alternate embodiments monitor 108 may be a portable unit.

Data communicated to (and processed by) monitor 108 may include or represent, without limitation: physiologic patient data; device status information; time and date information; alarm/alert status; and other information related to the operation; status, or condition of the patient, related to any of the devices within local infusion system 102, or related to local infusion system 102 itself. For example, such data may include or represent bolus information, basal information, or sensor information. Such data may also include or represent information entered by the patient, a caregiver, or another person having access to a local device or a network device 110, such as, without limitation: reminders; event markers (for meals, exercise, or the like); alarms; notifications; or the like.

As used here, a "data communication network" represents any number of physical, virtual, or logical components, including hardware, software, firmware, and/or processing logic configured to support data communication between an originating component and a destination component, where data communication is carried out in accordance with one or more designated communication protocols over one or more designated communication media. Communication hardware utilized by a data communication network may include a mechanically detachable unit such as an SDIO, a USB ready wireless module, or the like. For example, data communication network 112 may include, without limitation: a computer network such as a local area network or a wide area network; a pager network; a cellular telecommunication network; a cordless telephone system; an 802.11 network (WiFi); an 802.16 network (WiMAX); the Internet; IEEE P1901 BPL (Broadband over Power Lines); a hospital data communication network (WMTS or other); a home network, such as a home control network, a home security system, or a home alarm system; the public switched telephone network; a satellite communication network; or the like. In practice, network communications between monitor 108 and network devices 110 may be routed by two or more different types of data communication networks using known or proprietary network interfacing techniques.

In an embodiment of system 100, monitor 108 may be suitably configured to support the transmission of network communications to: a networked monitor device, such as a piece of hospital monitoring equipment; a portable computer, such as a laptop PC, a palmtop PC, or a tablet PC; a stationary computer, such as a desktop PC; a personal digital assistant, which may also be a portable email device; a smart phone, which may also be a portable email device; a wireless phone, such as a cellular phone or a cordless phone; one or more additional computing devices or databases; or the like. This feature allows monitor 108 to facilitate scheduled, automatic, or on-demand uploading of data to a computing device associated with a caregiver, a medical facility, etc. For example, monitor 108 may include a physical or software-implemented switch or button that enables a user to initiate the transmission of data from monitor 108 to a caregiver computing device, to a medical facility network, or the like. The above list of possible network devices 110 is not exhaustive, and an implementation of system 100 can be designed to accommodate network communication with other network systems, equipment, computing devices, components, and elements that are external to local infusion system 102.

Physiological characteristic sensor 106, infusion pump 104, and monitor 108 may be configured to transmit and receive local communications within local infusion system 102, where such local communications are transmitted and received in accordance with one or more specified local data communication protocols. For example, local communications may be exchanged between these local devices using one or more wireless data communication protocols (which may leverage RF, infrared, magnetic induction, or other wireless techniques) and/or using one or more wired data communication protocols. Local infusion system 102 may be flexibly configured such that any given local device can communicate with any other local device, and a communication link or path between two local devices may be unidirectional or bidirectional.

Infusion pump 104 is configured to deliver fluid, such as insulin, into the body of a user via, for example, an infusion set. In this regard, infusion pump 104 may have a replaceable or refillable fluid reservoir for the insulin, and the amount of fluid in the reservoir is considered to be an exhaustible operating quantity of infusion pump 104. In portable and personal implementations, infusion pump 104 may have a replaceable or rechargeable battery (or batteries) that provide operating power. The charge of the battery is also considered to be an exhaustible operating quantity of infusion pump 104. In practice, infusion pump 104 may have additional and/or alternative exhaustible operating quantities associated therewith.

In accordance with one exemplary embodiment, infusion pump 104 serves as a central hub that sends data to monitor 108. In some embodiments, the local medical device system need not include infusion pump 104, for example, monitoring systems utilized in conjunction with traditional insulin injection therapy. Moreover, infusion pump 104 need not include a display. In an embodiment that lacks a display, monitor 108 or any other device within local infusion system 102 may serve as a remote display for infusion pump 104. Other options for a remote display include, but are not limited to, any of the network devices 110 described above, e.g., a wireless phone, a portable computer, or a personal digital assistant. In practice, operation of infusion pump 104 may be remotely controlled by a remote control device (not shown) and/or by monitor 108. Control of infusion pump 104 may also be possible via a suitably configured user interface located at infusion pump 104 itself.

Local infusion system 102 may also include physiologic characteristic sensor 106, which is suitably configured to measure a physiologic characteristic of the patient. In addition, sensor 106 may include processing and control logic that enables it to control the operation of infusion pump 104. Such control may be responsive to measurements obtained by sensor 106. In the exemplary system described here, sensor 106 is a continuous glucose sensor that measures the glucose level of the patient in real time. Sensor 106 may include a wireless transmitter that facilitates transmission of physiological data of the user to other devices within local infusion system 102, such as infusion pump 104. Sensor 106 may also be linked to monitor 108 so that monitoring and programming of medication delivery may be performed remotely. Alternatively, sensor 106 may be configured to communicate directly with network devices 110 via, e.g., Bluetooth, ZigBee, or the like.

In practice, physiological characteristic sensor 106 may have a replaceable or rechargeable battery (or batteries) that provide operating power, and the charge of this battery is considered to be an exhaustible operating quantity of physiological characteristic sensor 106. Moreover, an embodiment of sensor 106 may have a limited lifespan and, therefore, sensor 106 may need to be periodically replaced. Thus, the replacement period associated with sensor 106 is also considered to be an exhaustible operating quantity of sensor 106. In practice, sensor 106 may have additional and/or alternative exhaustible operating quantities associated therewith. For instance, sensor 106 may be comprised of more than one component. Each sensor component may have one or more of the same or different exhaustible quantities. In a particular embodiment, sensor 106 is comprised of two separate components: a sensing element and an electronics package. The sensing element might have a shorter lifespan than the electronics package and, therefore, may need to be replaced more often than the corresponding electronics package.

For the illustrated embodiment, infusion pump 104 can process the received sensor data in an appropriate manner. For example, infusion pump 104 may display the current glucose level derived from the received sensor data and/or generate an alert or otherwise indicate low or high glucose levels. As another example, infusion pump 104 may process the received sensor data for purposes of calibration. Indeed, a calibration period associated with physiological characteristic sensor 106 (or infusion pump 104) can be considered to be an exhaustible operating quantity of sensor 106 (or infusion pump 104). As yet another example, infusion pump 104 may be configured to activate its infusion mechanism in response to the received sensor data. Moreover, sensor data could be processed in infusion pump 104, monitor 108, and/or in one or more of network devices 110. In this regard, system 100 may utilize distributed processing techniques for the handling of sensor data.

Any of the devices within local infusion system 102 may include a display and related processing logic that facilitates the display of physiologic patient data, device status information, time and date information, alarm/alert status, and other information related to the operation, status, or condition of the patient, related to any of the devices within local infusion system 102, or related to local infusion system 102 itself. In certain embodiments, monitor 108 and a network device 110 are in fixed locations in a home or building. In such embodiments, the system may also include a portable device (such as a necklace pendant, a clip-on device, a watch, a key fob, or the like) that generates alerts or alarms, displays sensor or pump data, or generates commands to control monitor 108 or infusion pump 104. This allows the patient or caregiver to move freely within the system environment. Moreover, an embodiment of system 100 may be configured to support wireless data communication from monitor 108, infusion pump 104, sensor 106 (or other devices) to eyewear that is suitably configured with near-eye display technology. Eyewear with near-eye displays would allow users to view information, such as alarms or alerts, quickly and without having to locate or manipulate a display device. A number of additional display features and characteristics of monitor 108 are described in more detail below.

In particular embodiments the infusion pump 104 is suitably configured to obtain BG meter data 118 from an appropriate source, such as a BG meter or test instrument (not shown) that measures the BG level of a user by analyzing a blood sample. For example, a BG meter may include a receptacle for receiving a blood sample test strip. In this regard, the user inserts a test strip into the BG meter, which analyzes the sample and displays a BG level corresponding to the test strip sample. The BG meter may be configured to generate a local communication, which conveys the measured BG level, for transmission to infusion pump 104. Alternatively or additionally, infusion pump 104 may include a user interface that allows the patient or caregiver to enter the measured BG level into infusion pump 104. Moreover, in certain embodiments of system 100 monitor 108 may be suitably configured to obtain BG meter data 118 in a similar manner.

Monitor 108, which may be realized as a bedside monitor for personal use or as a hospital monitor for caregiver use, enables remote monitoring of infusion pump 104 (and possibly other devices within local infusion system 102). Monitor 108 may be utilized in applications that do not utilize infusion pump 104; for example, applications that monitor patient data (such as glucose levels) without administering fluid to the patient. In such applications, monitor 108 can receive patient data directly from a sensor transmitter or a measurement device, or indirectly via an intermediary device, e.g., a patient-worn monitor or telemetry component. In yet another deployment, monitor 108 is utilized in a system that has an infusion pump, but does not have a sensor or meter for patient data. In such an application, monitor 108 receives, processes, and displays pump related data (where the pump data can be received directly from the infusion pump or indirectly from an intermediary device). Yet another embodiment of monitor 108 is used in a system having an infusion pump and a BG meter, but having no continuous glucose sensor transmitter. In such a system, monitor 108 can receive, process, and display pump related data and information related to BG meter readings, and monitor 108 will not display information related to a sensor transmitter. For the sake of completeness, the following description focuses on monitor embodiments that receive, process, and display pump related data and physiological patient data, namely, glucose data. In practice, these monitor embodiments may be self-reconfigurable to accommodate the type of transmitting device(s) and/or the type of data to be processed and displayed. For instance, if the source device is strictly a patient monitor, then monitor 108 may configure itself to display relevant monitor information (i.e., the pump related information, features, and functionality shown in the figures and described herein would neither be processed nor displayed). On the other hand, if the source device is strictly an infusion pump, then the monitor may configure itself to display pump related information (i.e., the glucose level information, features, and functionality shown in the figures and described herein would neither be processed nor displayed). In practice, monitor 108 may process distinguishable device codes or device identifiers (transmitted by the source devices) to determine the source device type and, in turn, to determine how best to reconfigure itself.

In addition, monitor 108 may be suitably configured to enable remote programming and control of infusion pump 104 and/or other devices within local infusion system 102. In this regard, a "monitor" as used herein can generally refer to a monitor-only device or a monitor-controller device. In practice, monitor 108 is a relatively large device in comparison to portable or handheld devices of local infusion system 102. In this regard, monitor 108 is intended to be somewhat stationary and not carried by the user. For example, a home monitor may be located on a nightstand beside the patient's bed, while a hospital monitor may be located on a medical equipment cart or stand in the patient's room. In contrast to the smaller portable devices of local infusion system 102, monitor 108 preferably includes a large and easy to read display element, which may be configured to concurrently reproduce at least a portion of the information displayed on infusion pump 104.

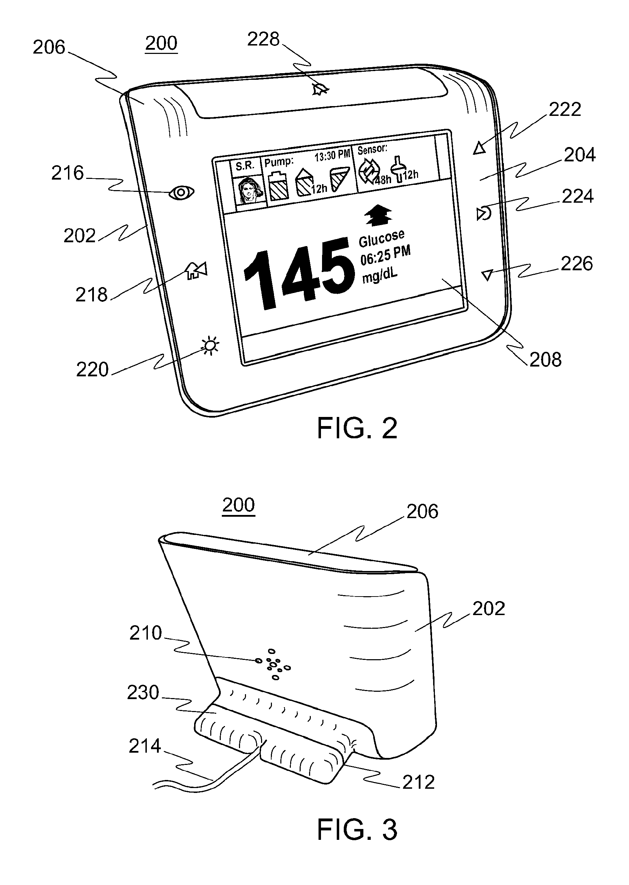

As described above, monitor 108 may also be configured to allow the user to remotely operate infusion pump 104. Thus, monitor 108 may include a local device interface for receiving and/or transmitting local communications within local infusion system 102. Moreover, monitor 108 may include a network interface for handling network communications to and from network devices 110 that are external to local infusion system 102. Further, monitor 108 may include one or more user input elements on its housing, such as keys, buttons, or the like, which accommodate user inputs. Embodiments of monitor 108 are described in more detail below with reference to FIGS. 2-4.

An embodiment of monitor 108 may also be capable of receiving, analyzing, and/or processing other data, which may be provided by one or more devices within the system. Such data may include, without limitation: body weight measurement data; blood pressure data; body temperature data; or the like. Once uploaded into monitor 108, this additional data can be handled and communicated in a manner equivalent to that described herein for infusion system data.

In addition, an embodiment of monitor 108 may be configured to recharge infusion pump 104, sensor 106, and/or other rechargeable devices in the system using wireless power transfer techniques and technologies. For example, monitor 108 could recharge a battery in infusion pump 104 while the patient is asleep, assuming that infusion pump 104 will be located within close proximity of monitor 108 during that time.

System 100 represents a simplified embodiment that stresses the functionality of monitor 108. Of course, monitor 108 and other devices and components in system 100 may be implemented and configured differently for use in other system or network architectures. For example, system 100 and the devices therein may employ the techniques, architectures, and technologies described in: United States patent application serial number 11/413,268, publication number _________; United States patent application serial number 11/583,344, publication number __________; and United States patent application serial number 11/671,174, publication number ____________; which are incorporated herein by reference.

FIG. 2 is a perspective front view of an embodiment of a monitor 200 suitable for use in a system such as that shown in FIG. 1, and FIG. 3 is a perspective rear view of monitor 200. Monitor 200 generally includes, without limitation: a housing 202; a face panel 204; a top panel 206; a display element 208; a speaker (or transducer) 210; a support structure 212; and a power cord 214. Monitor 200 is preferably sized for use at home on a nightstand, a reading table, a windowsill, or the like. For example, monitor 200 may have a height of about five inches, a width of about seven inches, and an overall depth of about three inches (including support structure 212). Alternatively, any of the features, functions, and operations of the monitors described herein may be supported by a monitor or other devices having different form factors, e.g., a portable monitor device, a PDA device, a computing device having monitor capabilities, or the like.

Housing 202, which may be an assembly of any number of parts, protects the internal components and electronics of monitor 200. Housing 202 may be formed from an appropriate material such as molded plastic, aluminum, or the like. In this embodiment, housing 202 functions as a support frame for face panel 204, top panel 206, and support structure 212. For example, face panel 204, top panel 206, and support structure 212 may be coupled to housing 202 using fasteners, adhesive, a press-fit engagement, or the like.

Face panel 204 and top panel 206 may be formed from glass, plastic, plexiglass, or the like. In preferred embodiments, face panel 204 and top panel 206 are formed from molded plastic. It should be appreciated that an embodiment of monitor 200 may utilize an integrated front panel that includes both face panel 204 and top panel 206 joined together in a seamless manner. The illustrated embodiment employs a face panel 204 that is flat except for its upper portion, which may be curved to match a curved contour of top panel 206. Face panel 204 may include a window area associated with display element 208 and an outer periphery surrounding the window area. The window area is clear to accommodate visibility of display element 208, while the outer periphery may be opaque, colored, or backed with a colored film.

Notably, one or more buttons, input/output elements, or other user interface features may be located at the outer periphery of face panel 204 and/or at top panel 206. For example, monitor 200 may include, without limitation, the following user interface elements: a view button 216; a home/back/left button 218; a night light on/off button 220; an up button 222; a forward/right button 224; a down button 226; and an alarm snooze button 228. For this embodiment of monitor 200, each of these "buttons" is implemented as a capacitive sensing element (rather than a physical switch, a mechanical button, a resistive flex panel button, or the like). In this regard, face panel 204 and top panel 206 need not include moving parts for any of these buttons, and the surfaces of face panel 204 and top panel 206 remain smooth and uninterrupted at and near these buttons. A user activates a capacitive sensing element by touching (or nearly touching) the outer surface of face panel 204 or top panel 206 at or near the respective button icon. For example, up button 222 may be tapped to advance through items of a displayed list, or it may be pressed and held to quickly scroll through the list. Monitor 200 may also incorporate capacitive sensing elements that support "slider" functionality: a user can manipulate a graphical user interface by moving his or her finger across the surface of face panel 204 or top panel 206 within a designated area.

In practice, the button icons depicted in FIG. 2 can be backlit for ease of operation. In preferred embodiments, monitor 200 selectively illuminates the button icons in response to its current operating state and/or in accordance with its currently available user interaction options. For example, when a monitor screen is displayed certain button icons, such as the icon for alarm snooze button 228, may not be illuminated. As another example, when an alarm is active and an alarm screen is displayed, it may be desirable to only illuminate the icons for alarm snooze button 228 and home/back/left button 218. Such selective and menu-driven backlighting of the button icons makes monitor 200 easier to operate because only those icons corresponding to currently operable buttons will be lit.

Alarm snooze button 228 may have a relatively large sensing area to accommodate quick and easy snoozing of alarms. On the other hand, alarm snooze button 228 may intentionally have a relatively small sensing area to reduce the likelihood that an alarm will be inadvertently snoozed and to reduce the likelihood that a user will snooze an alarm while asleep without paying attention to the alarm message. In certain embodiments, activation of alarm snooze button 228 only disables the audio component of an alarm or alert; the alarm message screen will remain on display element 208 until the use clears the alarm message. In particular embodiments, the alarm can only be cleared using the infusion pump or the patient-worn monitor device.

Display element 208, which may be incorporated into front panel 204, represents the primary graphical interface of monitor 200. Display element 208 may leverage known CRT, plasma, LCD, TFT, and/or other display technologies. For the illustrated embodiment of monitor 200, display element 208 includes a color monitor (for example, a thin film transistor display with CCFL backlighting, having a QVGA resolution of 320x240 or a VGA resolution of 640x480). Of course, the actual size, resolution, and operating specifications of display element 208 can be selected to suit the needs of the particular application. Notably, display element 208 and/or front panel 204 may be suitably configured as a touch screen that leverages known touch screen techniques and technologies such as "pinching," "grabbing," "zooming," and "rotating." In such an embodiment, monitor 200 need not include capacitive sense buttons.

Monitor 200 may also include one or more speakers or transducers 210. Speaker 210 is utilized to generate alarms, a startup jingle, reminder tones, and other audible indicia associated with the operation of monitor 200. Moreover, speaker 210 may be utilized to support media playback for monitor 200. For example, monitor 200 may be programmed with audio and/or video clips that are played for tutorials, instructions, or otherwise in connection with the operation of monitor 200. Speaker 210 can be used for the audio portion of such clips.

Support structure 212, which may be incorporated into housing 202, is suitably configured to support monitor 200 in a relatively upright position as depicted in FIG. 3 (in lieu of support structure 212, monitor 200 may be configured to accommodate wall mounting using fasteners, a wall bracket, or the like). Support structure 212 may be realized as a stand (as shown), a platform, a number of feet, etc.

In certain embodiments of monitor 200, support structure 212 incorporates or is realized as a night light 230. For example, support structure 212 may include a translucent plastic shell that houses one or more light elements. Night light 230 may configured to emit white light and/or colored light in a subdued manner that results in a soft and pleasant glow. Alternatively or additionally, night light 230 may be activated in a flashing mode to support alarm functions of monitor 200. Night light 230 may be particularly useful in embodiments where the main display is blanked after a period of time (for power saving) - night light 230 will enable the user to quickly locate monitor 200 in the dark. As mentioned above, night light on/off button 220 is used to control the activation of night light 230. In practice, the color, intensity, duration, and possibly other characteristics of night light 230 may be configurable and selectable by the user via the graphical user interface of monitor 200.

As shown in FIG. 3, power cord 214 may extend from behind monitor 200. Power cord 214 is compatible with a standard household AC power source, such as the standard 120 VAC supply available in the United States. In certain embodiments, monitor 200 does not include a main power on/off switch or button. Rather, monitor 200 is powered on by plugging power cord 214 in, and monitor 200 is powered off by removing power cord 214 from the source. This feature is desirable to ensure that monitor 200 remains continuously on after it is initialized. As described in more detail below, monitor 200 may include a backup power supply (e.g., a battery) that can be used if the primary power supply fails.

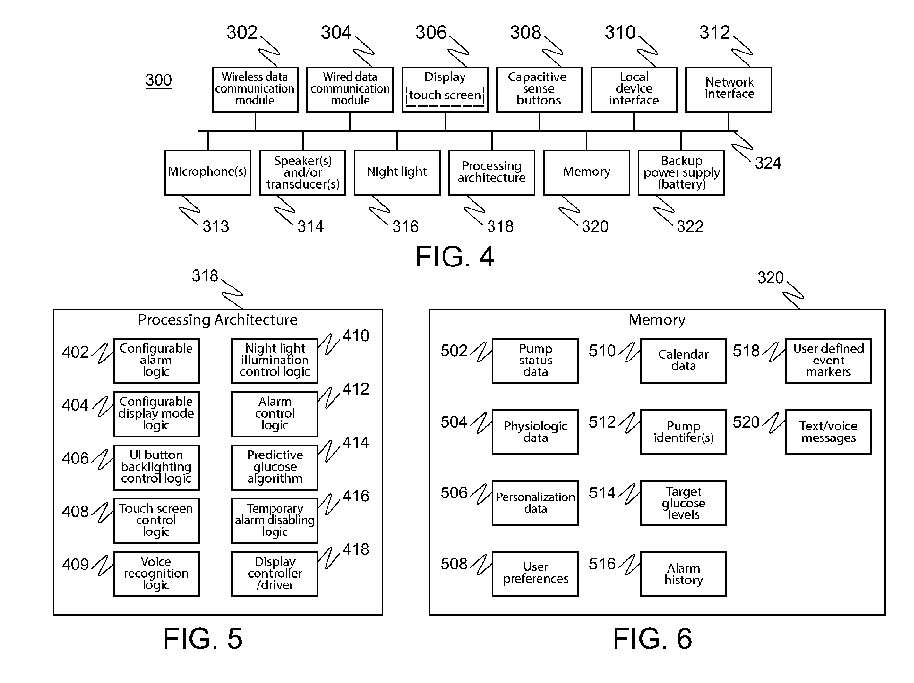

FIG. 4 is a schematic representation of an embodiment of a monitor 300. Monitor 108 (see FIG. 1) and monitor 200 (see FIG. 2) may employ some or all of the generalized architecture of monitor 300. For this example, monitor 300 generally includes, without limitation: a wireless data communication module 302; a wired data communication module 304; a display element 306; capacitive sense buttons 308; a local device interface 310; a network interface 312; one or more microphones 313; one or more speakers or transducers 314; a night light 316; a processing architecture 318; a suitable amount of memory 320; and a backup power supply 322. The elements of monitor 300 may be coupled together via a bus 324 or any suitable interconnection architecture.

Those of skill in the art will understand that the various illustrative blocks, modules, circuits, and processing logic described in connection with monitor 300 (and other devices, elements, and components disclosed here) may be implemented in hardware, computer software, firmware, or any combination of these. To clearly illustrate this interchangeability and compatibility of hardware, firmware, and software, various illustrative components, blocks, modules, circuits, and processing steps may be described generally in terms of their functionality. Whether such functionality is implemented as hardware, firmware, or software depends upon the particular application and design constraints imposed on the embodiment. Those familiar with the concepts described here may implement such functionality in a suitable manner for each particular application, but such implementation decisions should not be interpreted as causing a departure from the scope of the invention.

Display element 306 (with an optional touch screen), capacitive sense buttons 308, speakers/transducers 314, and night light 316 were described above in connection with monitor 200. Briefly, display element 306 may be suitably configured to enable monitor 300 to display physiological patient data, status information for infusion pumps, status information for continuous glucose sensor transmitters, clock information, alarms, alerts, and/or other information and data received or processed by monitor 300. For example, display element 306 may be controlled by a display controller/driver to indicate an alert or alarm status when monitor 300 receives an incoming communication (from a local device within the infusion system or from a network device external to the infusion system) that conveys an alert signal or an alarm signal. Capacitive sense buttons 308 enable the user to control the operation of monitor 300. In some embodiments, capacitive sense buttons 308 enable the user to control the operation of one or more additional devices within the local infusion system, for example, an infusion pump.

Processing architecture 318 may be implemented or performed with a general purpose processor, a content addressable memory, a digital signal processor, an application specific integrated circuit, a field programmable gate array, any suitable programmable logic device, discrete gate or transistor logic, discrete hardware components, or any combination designed to perform the functions described here. A processor may be realized as a microprocessor, a controller, a microcontroller, or a state machine. Moreover, a processor may be implemented as a combination of computing devices, e.g., a combination of a digital signal processor and a microprocessor, a plurality of microprocessors, one or more microprocessors in conjunction with a digital signal processor core, or any other such configuration.

In practice, processing architecture 318 may be suitably configured to interpret and process incoming information, data, and content that is conveyed in local communications received from a transmitting device (e.g., an infusion pump) within the local infusion system. Such incoming information may include, without limitation: physiological data of the user, such as a glucose level (a calibrated reading or a raw measured value); status information of the transmitting local device (e.g., a battery life indication, a power on/off status, an indication of the amount of fluid available for delivery, an estimation of time until the pump must be refilled with fluid, an estimate of time until a component, e.g., an infusion set, a reservoir, or a sensor, must be replaced, operating status such as whether the pump is in manual or automatic closed loop mode, a transmit signal power level, diagnostic information indicating results of self tests); an alert signal related to operation of the transmitting local device (e.g., a low battery alert, an out of range alert, a calibration reminder); a basal rate of fluid delivered to the user by an infusion pump; bolus information for a bolus of fluid delivered to the user by an infusion pump; advisory information for the patient (e.g., a notification to place an order for supplies, a reminder to schedule a doctor's appointment, a reminder to schedule or automatically execute a data download for analysis by a caregiver, a notification to perform routine diagnostics, either manually or remotely via a network connection); or the like.

Memory 320 may be realized as RAM memory, flash memory, EPROM memory, EEPROM memory, registers, a hard disk, a removable disk, a CD-ROM, or any other form of storage medium known in the art. In this regard, memory 320 can be coupled to processing architecture 318 such that processing architecture 318 can read information from, and write information to, memory 320. In the alternative, memory 320 may be integral to processing architecture 318. As an example, processing architecture 318 and memory 320 may reside in an ASIC.

An embodiment of monitor 300 may employ any number of wireless data communication modules 302 and any number of wired data communication modules 304. For simplicity, FIG. 4 only depicts one wireless data communication module 302 and one wired data communication module 304. These data communication modules 302/304 are suitably configured to support wireless/wired data communication (unidirectional or bidirectional, depending upon the particular implementation) between monitor 300 and other compatible devices. For example, a data communication module may be utilized to receive current status data of a medical device in the system (such as an infusion pump or a physiological characteristic sensor transmitter), where the current status data indicates a remaining measurement for an exhaustible operating quantity of the medical device. As another example, a data communication module may be utilized to send alarm/alert signals, messages, or packets (that contain or convey voice content, text content, or other information) to receiving devices. For the embodiment illustrated in FIG. 1, wireless data communication module 302 is configured to wirelessly receive the current status data from infusion pump 104 via wireless link 116.

Wireless data communication module 302 is configured to support one or more wireless data communication protocols. In practice, wireless data communication module 302 may be partially or completely realized in processing architecture 318. Any number of suitable wireless data communication protocols, techniques, or methodologies may be supported by monitor 300, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB. In an embodiment of monitor 300, wireless data communication module 302 may include or be realized as hardware, software, and/or firmware, such as an RF front end, a suitably configured radio module (which may be a stand alone module or integrated with other or all functions of the device), a wireless transmitter, a wireless receiver, a wireless transceiver, an infrared sensor, an electromagnetic transducer, or the like. Moreover, monitor 300 may include one or more antenna arrangements (which may be located inside the housing of monitor 300) that cooperate with wireless data communication module 302.

Depending upon its deployment, monitor 300 may cooperate with one or more wireless repeaters that function to extend the wireless transmission range of monitor 300. Moreover, monitor 300 itself may be configured to function as a wireless repeater for one or more other monitor devices. An embodiment of such a wireless repeater may be a compact device that can be plugged into a standard household wall outlet (similar to a compact powered air freshener or a nightlight), connected to a computing device via a USB port, or otherwise coupled to a power source. Alternatively, a wireless repeater may be battery powered. An embodiment of a wireless repeater may also include some of the functionality of monitor 300. For example, a wireless repeater may include a small display screen and/or a speaker or transducer for generating audible alerts. Accordingly, a suitably configured wireless repeater device may cooperate with an infusion pump (or other devices within the local device network) even in the absence of monitor 300.

Wired data communication module 304 supports data transfer over a cable, a wired connection, or other physical link. In practice, wired data communication module 304 may be partially or completely realized in processing architecture 318. Wired data communication module 304 is configured to support one or more wired/cabled data communication protocols. Any number of suitable data communication protocols, techniques, or methodologies may be supported by monitor 300, including, without limitation: Ethernet; home network communication protocols; USB; IEEE 1394 (Firewire); hospital network communication protocols; and proprietary data communication protocols. In an embodiment of monitor 300, wired data communication module 304 may include or be realized as hardware, software, and/or firmware, such as a suitably configured and formatted port, connector, jack, plug, receptacle, socket, adaptor, or the like.

An embodiment of monitor 300 may employ any number of local device interfaces 310 and any number of network interfaces 312. For simplicity, FIG. 4 only depicts one local device interface 310 and one network interface 312. Local device interface 310, which may be realized as hardware, software, and/or firmware, supports data communication between monitor 300 and devices within the personal infusion system, in particular, the infusion pump (see FIG. 1). In practice, local device interface 310 may be partially or completely realized in processing architecture 318. Local device interface 310 may cooperate with wireless data communication module 302 and/or wired data communication module 304 to send/receive data in a manner that is compatible with the particular data communication protocol. In this regard, local device interface 310 may be suitably configured for compatibility with one or more of the wireless and wired data communication protocols mentioned herein.

Network interface 312, which may be realized as hardware, software, and/or firmware, supports data communication between monitor 300 and network devices. In practice, network interface 312 may be partially or completely realized in processing architecture 318. Referring again to FIG. 1, network interface 312 may be suitably configured to facilitate data communication with data communication network 112 and with network devices 110. Network interface 312 may cooperate with wireless data communication module 302 and/or wired data communication module 304 to send/receive data in a manner that is compatible with the particular data communication protocol. In this regard, network interface 312 may be suitably configured for compatibility with one or more of the wireless and wired data communication protocols mentioned herein.

Backup power supply 322 provides backup operating power to monitor 300 in response to a failure condition of the primary power supply (e.g., the AC outlet supply voltage). Backup power supply 322 may be realized as a rechargeable or disposable battery. An embodiment of monitor 300 may be configured to generate an alert, a message, or to otherwise notify the user when backup power supply 322 is active. For example, monitor 300 may display an icon that represents operation using normal AC power and a different icon that represents operation using backup power supply 322.

FIG. 5 is a schematic representation of processing logic and/or modules that may be implemented in an embodiment of a monitor. For example, the processing logic and processing modules depicted in FIG. 5 may be associated with processing architecture 318 of monitor 300 (see FIG. 4). For this embodiment, the processing logic and processing modules include, without limitation: configurable alarm logic 402; configurable display mode logic 404; user interface (UI) button backlighting control logic 406; touch screen control logic 408; voice recognition logic 409; night light illumination control logic 410; alarm control logic 412; a predictive glucose algorithm 414; temporary alarm disabling logic 416; and a display controller/driver 418. The processing logic and processing modules represent various features, functions, and operations that might be supported by an embodiment of a monitor. These and other features, functions, and operations are described in more detail herein.

FIG. 6 is a schematic representation of data types and/or information that may be stored and processed by an embodiment of a monitor. For example, the data and information depicted in FIG. 6 may be stored in memory 320 of monitor 300 (see FIG. 4). For this embodiment, memory 320 stores some or all of the following data and information, without limitation: pump status data 502; physiologic data 504; personalization data 506; user preferences 508; calendar-related data 510; pump identifier(s) 512; target glucose levels 514; alarm history data 516; user-defined event markers 518; and text/voice messages 520. Alternate embodiments may store and process different data types. For example, an embodiment that does not process infusion pump data need not store pump status data 502, and an embodiment that does not process glucose data need not store physiologic data 504 or target glucose levels 514. As another example, an embodiment may support an infusion pump that provides BG meter readings obtained directly from the user or via telemetry with a BG meter (without any continuous glucose sensor data); in such an embodiment the physiologic data 504 represents the BG meter data and memory 320 need not store any information related to the status or operation of a continuous glucose sensor transmitter. In practice, the monitor uses the data and information maintained in memory 320 in connection with its features, functions, and operations. These and other data types and information elements used by an embodiment of a monitor are described in more detail herein.

General Operating Features

This section describes a number of general operating aspects and features of an embodiment of a monitor. For example, a number of display features and characteristics are described herein. In this regard, a monitor may utilize display controller/driver 418, which might be coupled to configurable display mode logic 404, processing architecture 318 and display element 306 (FIG. 4 and FIG. 5). Display controller/driver 418 may be suitably configured to perform image, graphics, and video processing as needed to support the operation of the monitor. For example, display controller/driver 418 may be employed to generate the various screens described herein, where the screens are rendered on display element 306.

The monitor also supports a number of alarm-related features and characteristics. Briefly, a monitor may utilize alarm control logic 412 to process and generate various alarms associated with the operation of itself, an infusion pump, a physiological characteristic sensor transmitter, or the like. Such alarm control logic 412 may be influenced by configurable alarm logic 402, configurable display mode logic 404, predictive glucose algorithm 414, temporary alarm disabling logic 416, user preferences data 508, target glucose levels 514, and/or other parameters and data types handled by the monitor (FIG. 5 and FIG. 6).

A monitor may also utilize voice recognition techniques and technologies to support a number of features and operations. Microphone 313 (FIG. 4) and voice recognition logic 409 (FIG. 5) can be utilized for voice recognition purposes. For example, the monitor can be configured to respond to voice commands in lieu of, or in addition to, commands initiated by user interaction with the monitor. Such voice commands can be utilized to traverse through menus, to activate features or functions of the monitor, to respond to alarms and alerts, etc. A monitor may also be configured to automatically respond in a predetermined manner if it does not receive anticipated voice commands. For example, if the monitor detects an emergency condition or state (e.g., low glucose) but does not detect an appropriate voice response from the user (e.g., "disable alarm" or "ignore"), then the monitor may automatically dial 911, attempt to contact the patient's caregiver, escalate the volume of the alarm, or the like.

Moreover, a monitor may include adaptive or dynamic characteristics that are associated with the patient's sleep cycles or sleeping patterns. The adaptive monitor characteristics may be, without limitation: volume; display brightness; nightlight activation and/or brightness; and alarm/alert style. Information related to the patient's sleep cycles or sleeping patterns may be derived in response to physiological patient data obtained by the monitor, or such information may be entered into the monitor by the patient. As one non-limiting example, the monitor may be set to generate relatively loud alarms during periods of deep sleep, and relatively quiet alarms during periods of light sleep. If the monitor is accurately calibrated with the patient's sleeping pattern, then adaptive alarms based upon the sleeping pattern can be utilized to reduce the number of missed nighttime alarms. In fact, alarms may be user-configurable such that the user can select which alarms are active during different times of the day.

Referring again to FIG. 1, the infusion system 102 may include two or more redundant sensors 106 for monitoring the same physiological characteristic. The use of redundant sensors 106 may be desirable to ensure that accurate measurements are obtained and to ensure continued operation if one of the sensors fails. If redundant sensors 106 are used, monitor 108 can be configured to indicate which sensor 106 is working better, which sensor 106 is transmitting a stronger signal, which sensor 106 is newer, etc. Monitor 108 can analyze such diagnostic and performance data for sensors 106 and, in response thereto, automatically select which sensor (or sensors) to use. Alternatively, monitor 108 may present the diagnostic data to the user and allow the user to select which sensor to use.

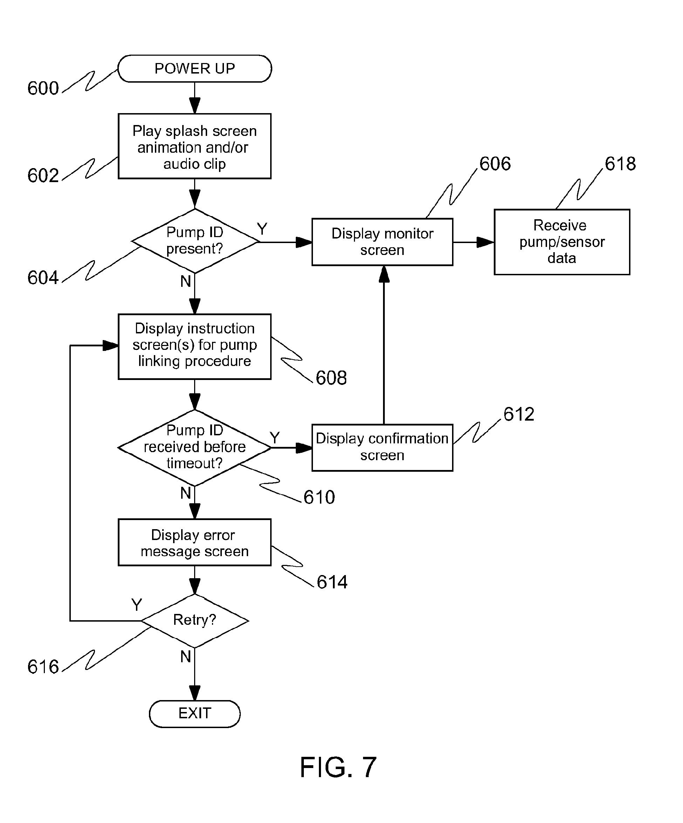

FIG. 7 is a flow chart that illustrates an embodiment of a power up process 600 for a monitor. Process 600 may be performed whenever the monitor is plugged in or otherwise powered on. The various tasks performed in connection with process 600 may be performed by software, hardware, firmware, or any combination thereof. For illustrative purposes, the following description of process 600 may refer to elements mentioned above in connection with FIGS. 1-6. In practice, portions of process 600 may be performed by different elements of the described system. It should be appreciated that process 600 may include any number of additional or alternative tasks, the tasks shown in FIG. 7 need not be performed in the illustrated order, and process 600 may be incorporated into a more comprehensive procedure or process having additional functionality not described in detail herein.

Process 600 may begin by playing a splash screen animation, a splash screen video clip, and/or an audio clip (task 602). The animation/video may identify the manufacturer of the monitor, and the audio clip may represent a jingle or a distinctive pattern of tones that is associated with the manufacturer of the monitor. The introductory sound clip may be desirable to reinforce branding of the monitor. When the monitor is powered up for the first time (or at any time thereafter at the request of the user), a setup wizard may be launched to provide step-by-step instructions for initializing the monitor. As described in more detail herein, the monitor may be provisioned with a number of user-configurable options, and the setup wizard may guide the user through the various optional settings.

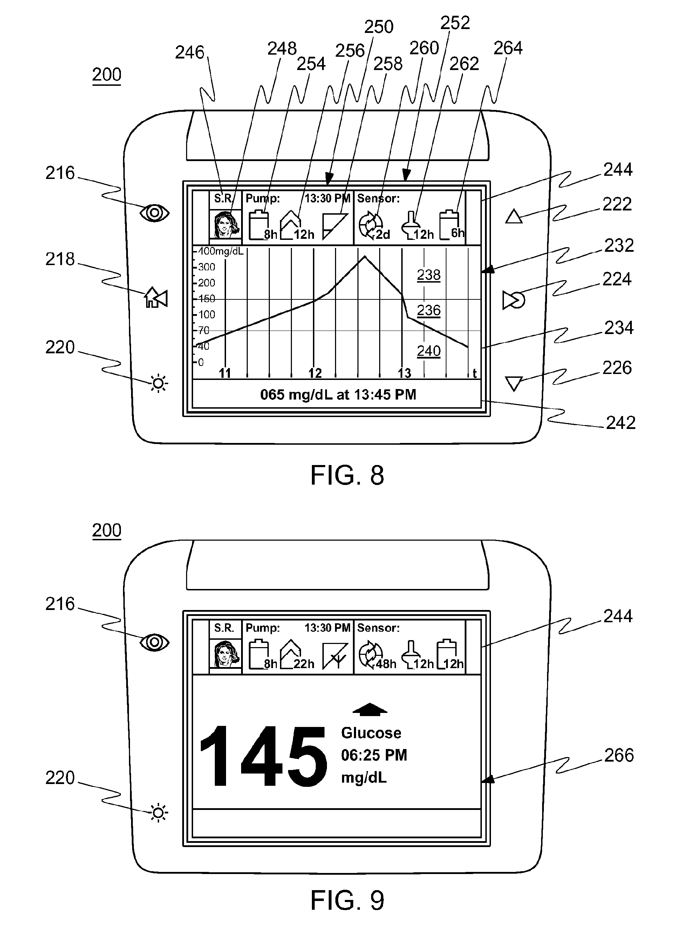

If a pump identifier is already present in the monitor (query task 604), then power up process 600 causes the monitor to display an appropriate monitor screen (task 606). In certain embodiments, process 600 may prompt the user to enter a password before the monitor screen is displayed. Password protection may be desirable to maintain the privacy of the user and/or to prevent unauthorized tampering of the monitor. The monitor screen, which may be user-selectable, represents the "default" or main screen for a linked infusion pump. In this regard, FIG. 8 and FIG. 9 depict front views of monitor 200 having different monitor screens displayed thereon. The monitor screen depicted in FIG. 8 includes a graph of glucose level versus time, while the monitor screen depicted in FIG. 9 includes the current numerical value of the glucose level, along with an arrow (pointing up in this example) that indicates a rising trend in the glucose level. The content of these monitor screens is described in more detail below.

If a pump identifier is not present (query task 604) or if the monitor is not linked with the particular infusion pump, which may occur in embodiments that support multiple infusion pumps, then power up process 600 causes the monitor to display one or more instruction screens for a pump linking procedure (task 608). These instruction screens may provide written, audio, and/or video content to guide the user through the pump linking procedure. For example, the instruction screens may instruct the user to place the infusion pump in close proximity to the monitor to accommodate wireless data communication. The instruction screens may also provide specific instructions related to manipulation of the infusion pump for purposes of the linking procedure. If the monitor receives the pump identifier before a timeout period has elapsed (query task 610), then process 600 causes the monitor to generate and display a confirmation screen (task 612). In practice, the received pump identifier will be saved by the monitor. In this regard, FIG. 6 depicts pump identifier(s) 512 stored in memory 320. Following task 612, the monitor can switch (either automatically or in response to a user request to do so) to display the monitor screen (task 606).

If the pump identifier is not received within the timeout period (e.g., five minutes), then power up process 600 may cause the monitor to generate and display an appropriate error message screen (task 614). For example, the error message screen may inform the user that the linking procedure failed, and instruct the user to try again or contact a support technician for assistance. If the monitor is prompted to retry the linking procedure (query task 616), then process 600 may be re-entered at, for example, task 608. Otherwise, process 600 may exit. Similarly, in particular embodiments the monitor may be linked with other devices such as a sensor, BG meter, wireless repeaters, remote portable displays, a remote controller, and the like. In particular embodiments, once a device is linked with the monitor, all other devices in communication with the monitor receive information so that they are linked with the device as well.

After the monitor has been initialized with the infusion pump, it may then receive pump and/or sensor data in an ongoing manner (task 618). Initially, some historical pump data, the target glucose range of the patient, or pump settings may be transferred from the infusion pump to the monitor. In this regard, FIG. 6 depicts pump status data 502, physiologic data 504, target glucose levels 514, and alarm history data 516 stored in memory 320. If wireless connectivity is maintained between the monitor and the infusion pump, then the pump data can be wirelessly transmitted to the monitor in accordance to a desired schedule, for example, once every five minutes. If wireless connectivity is lost, then the infusion pump may batch download accumulated data to the monitor after wireless connectivity has been reestablished.

Although an embodiment of a monitor may be suitably configured to receive data directly from a continuous glucose sensor transmitter, the example described here assumes that such sensor data is transmitted from the sensor transmitter to the infusion pump, which then forwards the sensor data to the monitor. In this regard, "pump data" as referred to here may also include sensor data.

Graphical User Interface Features

A monitor as described here can generate a number of display screens having different graphical user interface (GUI) features, icons, display elements, and the like. A number of display screens and GUI features will be described below with reference to monitor 200. Of course, these and other GUI features can also be utilized in alternate embodiments of a monitor. Notably, FIGS. 2 and 8-18 depict sample display screens produced by a monitor that handles infusion pump data and glucose data. As mentioned previously, the display screens and functionality of an embodiment of monitor 200 may differ than that shown and described here, depending upon the transmitting source device type and the specific data type handled by monitor 200. For example, if monitor 200 does not support infusion pumps or if the patient does not use an infusion pump, then the pump status icons and pump related alarm features described here can be eliminated. On the other hand, if monitor 200 does not support continuous glucose sensor transmitters or if the patient does not use such sensor transmitters, then the sensor status icons and sensor transmitter related alarm features described here can be eliminated. Indeed, in certain embodiments monitor 200 can reconfigure itself as needed to provide a feature set and display characteristics that correspond to the transmitting source device(s).

FIG. 8 depicts one possible monitor screen 232 for monitor 200. As mentioned briefly above, monitor screen 232 represents a default or primary display for monitor 200, which can be updated whenever monitor 200 receives new pump data. Monitor screen 232 includes a graph 234 of the patient's glucose level over time, where the rightmost end of the plot represents the current glucose level. Graph 234 preferably includes indicia of the patient's target glucose range. For example, graph 234 may include a target zone 236, a hyperglycemic zone 238, and a hypoglycemic zone 240, where these zones have distinguishable colors, patterns, brightness, or other characteristics. In this regard, target zone 236 may be colored green, hyperglycemic zone 238 may be colored orange, and hypoglycemic zone 240 may be colored red. In addition to graph 234, other graphs and/or non-graphical information can be concurrently displayed here, e.g., graphs related to basal pattern, insulin-on-board, or the like. Notably, a particular embodiment of monitor 200 is also capable of processing and displaying BG meter readings obtained directly from a BG meter or indirectly via an intermediary device such as an infusion pump or patient-worn monitor. Although the various display screens shown in the figures are associated with calibrated glucose values, it may also be desirable to process and display information related to the BG meter readings, e.g., the actual readings, plots/graphs of the readings, the number of readings taken during a specified period of time, the time of the readings, and various alerts and reminders associated with BG meter readings. It should be appreciated that the generalized display features and operations described here for calibrated glucose values can also be extended for equivalent use in connection with the display of BG meter readings.

Monitor screen 232 may also include a text field 242 that can be used to display information such as the current glucose level and the time at which the current glucose level was measured. As an example, text field 242 in FIG. 8 shows a glucose level of 65 mg/dL, taken at 13:45 PM. Monitor screen 232 may also include a number of graphical icons that represent different operating conditions, status, settings, preferences, etc. For this particular embodiment, these icons are located at the top of monitor screen 232, in an icon bar 244. Icon bar 244 may include, without limitation: the name or initials 246 of the user; an avatar/image 248 of the user; a pump field 250; and a sensor field 252. Pump field 250 may include a pump battery status icon 254, a pump reservoir status icon 256, and a signal strength icon 258. Sensor field 252 may include a sensor calibration timer icon 260, a sensor life timer icon 262, and a sensor battery status icon 264. Of course, an embodiment of monitor 200 need not display all of the icons shown in FIG. 8. Moreover, an embodiment of monitor 200 may display additional or alternative icons. Any of these icons may be displayed in a flashing manner or in a specific color scheme that indicates a "low" or "warning" condition prior to the generation of an alarm. In certain embodiments, if icon data is not available (which may happen if a device is missing or is disconnected), then monitor 200 displays a "no data" icon or text in the appropriate location.

Monitor 200 may enable the user to personalize the display with a graphical representation of personalization data 506, which may include, without limitation: image data, avatar data, user initials data, user name data, digital photograph data, graphics data, and/or alphanumeric data. For example, display controller/driver 418 may be configured to generate graphical elements that correspond to the user's name or initials 246 and/or an avatar/image 248. In this regard, monitor 200 may generate a menu-driven display screen that enables the user to enter and save his or her name or initials 246 using the capacitive sensing buttons described above. Referring to FIG. 6, name or initials 246 may be stored as part of personalization data 506 in memory 320. Avatar/image 248 may also be stored as part of personalization data 506 in memory 320. Avatar/image 248 may be a photograph, a cartoon, a drawing, a symbol, or any graphical item. The data for avatar/image 248 may be saved in any suitable format, such as a JPEG file, a bitmap file, or the like. In some embodiments of monitor 200, the data for avatar/image 248 is "canned" data that is pre-loaded into memory 320 during manufacture, and the user is able to select one of the pre-loaded images for use as avatar/image 248. Other embodiments of monitor 200 may be suitably configured to accommodate uploading of data for avatar/image 248 using, for example, wireless data communication module 302, wired data communication module 304, or a portable memory device (see FIG. 4). In such embodiments, avatar/image 248 may represent a digital photograph of the user or any image selected by the user.

As mentioned above, an infusion pump may be powered by a battery that has an exhaustible charge. Here, monitor screen 232 includes a status icon that graphically indicates the remaining charge time for the infusion pump battery. Pump battery status icon 254 graphically indicates the current charge status of the battery in the respective infusion pump. For example, a fully colored battery icon might indicate a full battery, while a half-colored battery icon might indicate a partially charged battery. Moreover, pump battery status icon 254 may include an alphanumeric time indicator that shows the approximate amount of charge time remaining on the pump battery. For example, FIG. 8 indicates that about eight hours of charge remain on the pump battery. In certain embodiments, pump battery status icon 254 may initially indicate the approximate number of days that remain if the pump battery has at least one full day of charge. When less than a full day remains, pump battery status icon 254 switches to display the number of hours that remain. This feature allows the user to better manage system alerts. For example, if before going to sleep the user notices that only three hours of pump battery life remain, then the pump battery can be replaced or recharged to ensure that a low battery alert is not activated overnight.

As mentioned above, an infusion pump may include a refillable or replaceable reservoir that has an exhaustible supply of fluid. Here, monitor screen 232 includes a status icon that graphically indicates the remaining volume of fluid in the reservoir. Pump reservoir status icon 256 graphically indicates the amount of fluid (e.g., insulin) that remains in the infusion pump reservoir. For example, a fully colored reservoir icon might indicate a full reservoir, while a slightly colored reservoir icon might indicate a reservoir that is almost empty. Additionally or alternatively, pump reservoir status icon 256 may include an alphanumeric volume indicator that shows the current volume (in an appropriate scale such as mL) of fluid remaining in the reservoir. Additionally or alternatively, pump reservoir status icon 256 may include an alphanumeric indicator that shows the number of insulin units remaining in the reservoir. Moreover, pump reservoir status icon 256 may include an alphanumeric time indicator that shows (in days and/or hours) when the pump reservoir will be empty. For example, FIG. 8 indicates that the pump reservoir will be empty in about twelve hours. As described above for pump battery status icon 254, pump reservoir status icon 256 may switch scales from days to hours as needed. Again, this feature allows the user to better manage system alerts.

Signal strength icon 258 graphically indicates the strength of the wireless signal received from the infusion pump. For example, a fully colored icon might indicate relatively high signal strength, while a slightly colored icon might indicate little or no signal strength. The underlying data for signal strength icon 258 may be related to a quality of service characteristic calculated by wireless data communication module 302 (see FIG. 4) or by another processing element of monitor 200.

In practice, the raw physiological data obtained from a continuous glucose sensor transmitter is calibrated against actual BG measurements obtained from a blood sample. Blood strips or sticks are typically utilized for such calibrations. In this embodiment, monitor screen 232 includes a status icon that graphically indicates the remaining time until the current calibration ends, the time until the next calibration must be performed, etc. For example, sensor calibration timer icon 260 graphically indicates the time remaining until the next sensor calibration is due. For example, a fully colored calibration icon might indicate that a calibration was recently performed, while a slightly colored calibration icon might indicate that the next calibration must be performed soon. Moreover, sensor calibration timer icon 260 may include an alphanumeric time indicator that shows the approximate amount of time remaining (in days or hours) until a calibration is due. For example, FIG. 8 indicates that the next calibration is due in two days. As described above for pump battery status icon 254, sensor calibration timer icon 260 may switch scales from days to hours as needed. Again, this feature allows the user to better manage system alerts.

Continuous glucose sensors have a limited lifespan. Current state of the art continuous glucose sensors have a typical lifespan of about three days (even though the transmitter section may be rechargeable), and future sensors may have a longer lifespan. For this embodiment, monitor screen 232 includes a status icon that graphically indicates the remaining time until the replacement period ends, the time until the current sensor must be replaced, etc. In FIG. 8, sensor life timer icon 262 graphically indicates the time remaining until the continuous glucose sensor is due for replacement. For example, a fully colored sensor transmitter icon might indicate that the sensor was recently replaced, while a slightly colored sensor transmitter icon might indicate that the sensor must be replaced soon. Notably, sensor life timer icon 262 may also include an alphanumeric indicator that shows the approximate amount of time remaining (in days or hours) until the sensor must be replaced. For example, FIG. 8 indicates that a new sensor should be deployed in about twelve hours. As described above for pump battery status icon 254, sensor life timer icon 262 may switch scales from days to hours as needed. Again, this feature allows the user to better manage system alerts.

As described above, a physiological characteristic sensor transmitter may be powered by a battery that has an exhaustible charge. Thus, monitor screen 232 may include a status icon that graphically indicates the remaining charge time for the sensor transmitter battery. In FIG. 8, sensor battery status icon 264 graphically indicates the current charge status of the battery in the respective continuous glucose sensor transmitter. For example, a fully colored battery icon might indicate a full battery, while a half-colored battery icon might indicate a partially charged battery. Moreover, sensor battery status icon 264 may include an alphanumeric time indicator that shows the approximate amount of charge time remaining on the transmitter battery. For example, FIG. 8 indicates that about six hours of charge remain on the transmitter battery. As described above for pump battery status icon 254, sensor battery status icon 264 may switch scales from days to hours as needed. Again, this feature allows the user to better manage system alerts.

As stated previously, the monitor may maintain information related to the user's sleeping patterns. In this regard, a sleeping pattern for a user may indicate a normal bedtime and a normal waking time for that user. In certain embodiments, the monitor will generate an alert before (and preferably near) the user's bedtime if the monitor predicts that one or more exhaustible operating quantities will expire or become depleted before the user's normal waking time. For example, if the pump battery, the sensor battery, the sensor lifespan, or the amount of insulin in the pump reservoir will be depleted during the night, then the monitor will alert the user before bedtime. Furthermore, the monitor may be suitably configured to check other parameters near the user's bedtime, such as glucose level, the glucose rate of change, predicted glucose levels, how soon the next BG meter reading is needed for calibration, the amount of insulin on-board (i.e., the amount of active insulin in the user's body), and the like.

For any of the status icons described herein, monitor 200 may generate an animation and/or a graphical element that overlaps or temporarily replaces the normally rendered icon to indicate certain conditions, operating status, or the like. For example, monitor 200 may generate an appropriate animation for pump reservoir status icon 256 to indicate that the pump will administer a bolus and/or a basal dose of fluid. This feature provides a relatively real-time indication when the pump has been activated. As another example, monitor 200 may generate an appropriate animation for pump battery status icon 254 to indicate that the battery is currently being recharged.

Notably, the data rendered in pump battery status icon 254, pump reservoir status icon 256, sensor calibration timer icon 260, sensor life timer icon 262, and sensor battery status icon 264 may be included with the pump status data 502 received from the infusion pump (see FIG. 5). Thus, monitor 200 need not include timers or processing intelligence associated with the calculation of the time periods associated with the remaining pump battery charge, the remaining reservoir level, the sensor calibration period, the sensor replacement period, or the remaining sensor transmitter battery charge.

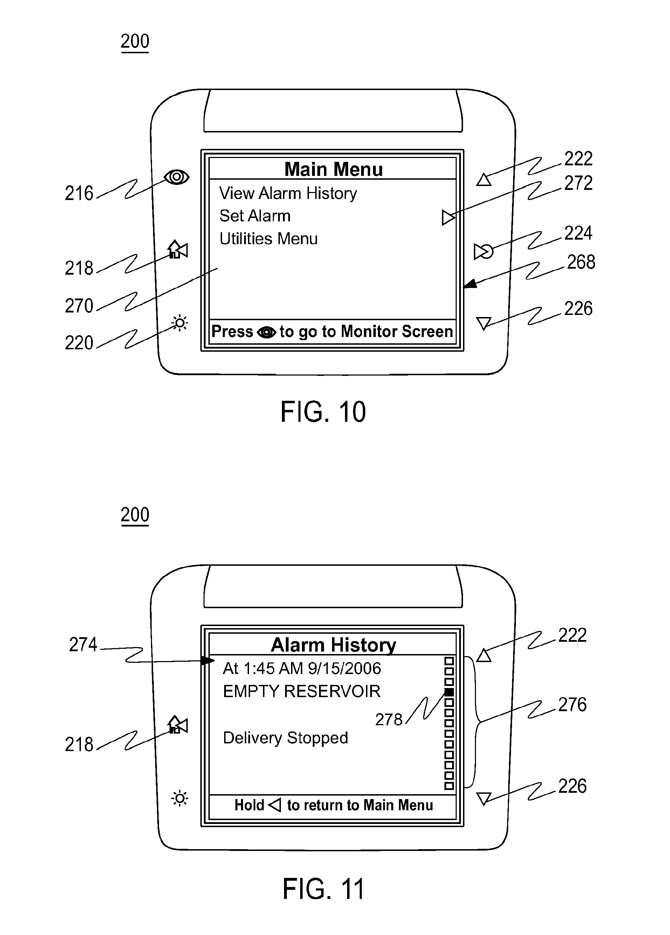

As mentioned above with reference to FIG. 2, monitor 200 controls the backlighting of the button icons on its front and top panels such that only currently functional button icons are illuminated. Monitor 200 may utilize backlighting control logic 406 (FIG. 5) to selectively illuminate these button icons. In this regard, FIG. 8 depicts a state of monitor 200 where view button 216, home/back/left button 218, night light on/off button 220, up button 222, forward/right button 224, and down button 226 are backlit, and all remaining button icons are dimmed or unlit. Up button 222 and down button 226 may be available here to control a graph zoom function that changes the displayed time range of the graph (e.g., three hours as shown in FIG. 8, six hours, twelve hours, a day, etc.). Home/back/left button 218 and forward/right button 224 can be used to move a vertical indicator line that corresponds to the currently displayed glucose level. View button 216 can be used to cycle through different types of monitor screens, along with a main menu screen (see FIG. 10) for monitor 200. For this embodiment of monitor 200, the monitor screens, along with the main menu screen, represent the upper display level of monitor 200. View button 216 allows the user to advance through the different monitor screens and the main menu screen until a desired screen is displayed. In this manner the user can set his or her desired "home" or "default" screen which will remain designated until it is changed by the user.

If monitor 200 supports more than one local medical device (which may be associated with one or more patients), then the user can be given the option of selecting a device or a patient for current monitoring by monitor 200. For example, the selection of a device or a patient may be accomplished via a submenu item listed on the main menu screen. Alternatively, it may be possible to utilize home/back/left button 218 and forward/right button 224 to select a currently monitored device while a monitor screen is displayed. For example, if monitor 200 supports three infusion pumps for three patients, then monitor 200 will illuminate home/back/left button 218 and forward/right button 224 on all monitor screens. Name/initials 246 and avatar/image 248 will change to identify the selected patient. On the other hand, if monitor 200 only supports one local medical device, then home/back/left button 218 and forward/right button 224 need not be backlit (alternatively, home/back/left button 218 may be utilized as a shortcut button for the main menu screen).

Certain embodiments of monitor 200 support the simultaneous display and handling of information for more than one patient/user. In this regard, multiple glucose values and/or multiple small-scale glucose graphs can be displayed together on the same screen, with appropriate identifiers (such as names or avatars) that indicate the respective patients. For example, the graph depicted in FIG. 8 may be reformatted to approximately half its size for display on a screen that simultaneously monitors two patients. Whether or not monitor 200 generates such multiple displays can be automatically determined in response to the initialization of multiple devices, or manually selected by the user of monitor 200.

FIG. 9 depicts another possible monitor screen 266 for monitor 200. In contrast to monitor screen 232 (FIG. 8), which displays a glucose level graph, monitor screen 266 displays the current glucose level in numerical form, along with the time corresponding to the current glucose level. Monitor screen 266 may also display an upward or downward pointing arrow to indicate whether the glucose level is trending upward or downward, respectively (single or double arrows can be displayed depending upon the rate of change). Alternatively, an arrow can point at different angles/directions, or an arrow can have different lengths, to indicate the rate of rising or falling glucose. The contents of icon bar 244 and the functionality of the various front panel buttons are as described above for monitor screen 232. Yet another monitor screen of monitor 200 may only include icon bar 244 and its associated content. Such a monitor screen may used when it is desirable to keep the patient's glucose levels private. A simplified monitor screen may be blank - icon bar 244 is not included in this simplified monitor screen. Monitor 200 may also use a blank monitor screen for a power saving mode that is automatically triggered if no activity occurs for a certain period of time. The monitor may display one or more pictures or an animated screen saver that is automatically or manually triggered if no activity occurs for a certain period of time. Moreover, the monitor may employ a motion detector to detect the presence of a user and, in response to detected motion, reactivate an appropriate data display.

FIG. 10 depicts a main menu screen 268 for monitor 200. As mentioned above, main menu screen 268 is considered to be part of the upper display level of monitor 200 (along with the various monitor screens). This embodiment of main menu screen 268 includes a scrollable list 270 corresponding to any number of selectable entries. Here, scrollable list 270 includes three entries: View Alarm History; Set Alarm; and Utilities Menu (of course, an embodiment of monitor 200 may include additional and/or alternative main menu entries). The currently selected item may be highlighted with an arrow 272, with a different color or pattern, with a different font, with a different brightness, or the like.

As described above with reference to FIG. 8, the user can switch from main menu screen 268 to a monitor screen by pressing view button 216. While main menu screen 268 and other menu screens are displayed, forward/right button 224 can be used to select the currently highlighted item in scrollable list 270, while home/back/left button 218 can be used to display a previous menu or as a shortcut back to main menu screen 268. In this regard, the operation of home/back/left button 218 and forward/right button 224 may be reconfigurable from one display screen to another. While main menu screen 268 and other menu screens are displayed, up button 222 and down button 226 can be used to scroll up and down, respectively, through the currently displayed list of items.

As mentioned above, main menu screen 268 includes a selectable item titled View Alarm History. FIG. 11 is a front view of the monitor shown in FIG. 2, with an alarm history screen 274 displayed. Alarm history screen 274 provides a GUI in the form of a selectable list of alarms corresponding to alarm history data 516, which may be stored in memory 320 (FIG. 6). In this embodiment, alarm history screen 274 includes a count/selection graphic 276 that represents the number of alarms contained in the history (i.e., the alarm count). Count/selection graphic 276 also serves as a scrollable selection item that enables the user to select an alarm to be currently displayed using up button 222 and down button 226. Thus, a count/selection graphic 276 having three markers (e.g., dots, lines, numerals, letters, or the like) indicates that the history contains three alarms. FIG. 11 depicts a scenario where the alarm history includes thirteen alarms. In one embodiment, alphanumeric characters are displayed next to the markers to specify the total number of alarms in the history - for example, "1/13" can be displayed next to the first marker, "2/13" can be displayed next to the second marker, and so on. If alarm history screen 274 cannot accommodate the number of alarms contained in the history, then up button 222 and down button 226 can be used to scroll through multiple pages as needed.

FIG. 11 depicts a state where the fourth alarm in the history has been selected for current viewing. As an example, the fourth dot 278 may be highlighted with a different color, brightness, pattern, size, or the like to indicate that the currently displayed alarm information corresponds to the fourth alarm in the history. Moreover, the markers in count/selection graphic 276 may be displayed using different colors, patterns, sizes, brightness, or the like to indicate the relative priority or urgency of the alarms. For example, red colored markers may indicate relatively urgent alarms, while yellow colored markers may indicate relatively low priority alerts or reminders.

Alarm history screen 274 may also display data or information related to the currently selected alarm. For example, the alarm data may include, without limitation: the time of the alarm; the date of the alarm; the type or cause of the alarm; user instructions for handling the alarm; or the like. FIG. 11 shows alarm history data for an Empty Reservoir alarm that occurred at 1:45 AM on September 15, 2006. Alarm history screen 274 also includes a brief note that states "Delivery Stopped." In this embodiment, alarm history screen 274 also instructs the user to hold home/back/left button 218 to return to main menu screen 268 (FIG. 10).

Referring again to FIG. 10, the "Set Alarm" item of main menu screen 268 may lead to a submenu list that allows the user to configure alarms generated by monitor 200. The Set Alarm submenu may enable the user to select different alarm tones or patterns, adjust alarm volumes, set alarm durations, prioritize alarm types, configure how alarms are handled, create voice message content and/or text message content for alarm/alert messages transmitted by monitor 200, and perform other alarm-related functions using the front panel buttons of monitor 200. A number of user-definable and user-selectable alarm and alert features are described in more detail below.

The "Utilities Menu" item of main menu screen 268 may lead to a submenu list that allows the user to perform various operations. The Utilities Menu screen itself may contain a list of selectable items. The selectable utilities items may include the any of the following, without limitation: Enter Pump Number; Set Language; Set Time; Set Units; Enter User Initials; Select User Image; Setup Network; View Tutorials; Set Night Light; Set Brightness; Calendar; and Upload/Download Data to or from PC (or other device). As described above with reference to alarm history screen 274, it may be possible to scroll through the selectable utilities entries using up button 222 and down button 226. In certain embodiments the date and time maintained by the monitor are automatically set via an appropriate signal, such as a satellite signal, a cellular signal, a synchronization signal from a computer network, a radio or television broadcast signal, or the like.

The submenu item "Enter Pump Number" launches a display screen that allows the user to manually enter the infusion pump number (or other identifier). This submenu item can be used to add other pumps or multiple users. Up button 222 and down button 226 may be configured to allow the user to scroll through the possible alphanumeric characters of the pump number, and home/back/left button 218 and forward/right button 224 may be configured to allow the user to select the current character position subject to scrolling. After the last digit of the pump number has been entered, a confirmation screen may be generated. The confirmation screen may display the entered pump number along with a prompt to hold home/back/left button 218 to return to the main menu. In practice, the manually entered pump number may be saved as a pump identifier 512 in memory 320 (FIG. 6). This general procedure may also be followed for the Set Language submenu item (to enable the user to select the language used by monitor 200), the Enter User Initials submenu item (to enable the user to input initials or a name), the Set Night Light submenu item (to enable the user to activate and/or adjust the brightness of night light 230), and the Set Brightness submenu item (to enable the user to adjust the overall brightness of display element 208).

The submenu item "Select User Image" launches a display screen that allows the user to select, create, or download an avatar, picture, or graphic for avatar/image 248 (FIG. 8). Alternatively, this function may be accessed via the Enter User Initials submenu item. This feature may enable the user to scroll through a number of preloaded images and avatars using the capacitive sense buttons on the front panel. Alternatively, this feature may provide instructions for transferring an image file to monitor 200 using a data communication link, a portable memory device, or the like. Ultimately, the selected image can be saved as personalization data 506 in memory 320 (FIG. 6).

The submenu item "Setup Network" launches a display screen that allows the user to configure the data communication network for monitor 200. This function assists the user in setting up monitor 200 and any wireless repeaters in the system environment, e.g., a home. In certain embodiments, this function may be associated with a setup wizard that provides step-by-step instructions for setting up the network. For example, this function may instruct the user to locate monitor 200 in the desired spot and test the maximum range (with or without repeaters). This could be accomplished by sending a continuous test message from a repeater while transporting it away from monitor 200. If an "out of range" indicator or alert is generated, the maximum range was determined and the repeater should be plugged into the nearest outlet towards monitor 200. This function may also help the user in determining how many repeaters are necessary to obtain the full desired coverage. Navigation of the various display screens for this feature may be accomplished using the capacitive sense buttons on the front panel.

The submenu item "View Tutorials" launches a display screen that allows the user to select tutorial audio clips, tutorial video clips, or written instructions related to the operation of monitor 200, the operation of the infusion pump, the operation of the continuous glucose sensor transmitter, the operation of the medical device system, or the like. In this regard, monitor 200 may employ "help desk" or troubleshooting scripts (which may include textual, audio, or video content) related to the operation of monitor 200. These scripts can be authored in a user-friendly manner such that the user need not contact a product support person or deal with a frustrating telephone-based voice response system. As another example, monitor 200 may employ network setup tutorials that assist the user when initializing and configuring the system. A network setup tutorial may provide instructions for locating monitor 200, wireless repeaters, and other wireless communication devices, and instructions for testing the wireless range of the various devices. In practice, monitor 200 may utilize an audible signal strength indicator and/or audio messages to instruct the user during the network setup; this feature allows the user to position the network devices and confirm their wireless ranges without having to walk back and forth between monitor 200 and the particular installation areas. Navigation of the various display screens for the tutorials and scripts may be accomplished using the capacitive sense buttons on the front panel. In particular embodiments, when an alarm is displayed on the monitor, the monitor also displays an option to play a tutorial related to the alarm. For example, if the monitor generates an "Empty Reservoir" alarm, the user has the option to play an audio or audio-visual tutorial that explains how to change the reservoir.

The submenu item "Calendar" launches a display screen that shows the current month, week, or day. Navigation of the various display screens for the calendar may be accomplished using the capacitive sense buttons on the front panel. For example, the user can highlight a day of the month and press the forward/right button 224 to show the details of the selected day. The calendar feature may accommodate the creation, editing, and deletion of appointments, reminders, and tasks, and it may include any number of features common to electronic calendar and scheduling applications. When in the month or week viewing mode, days with scheduled events may be highlighted using different colors, brightness, patterns, or the like. An embodiment of monitor 200 may also be suitably configured to maintain and display the current time, thus additionally serving as an alarm clock for the user. Referring to FIG. 6, the data associated with the calendar and scheduled events can be saved in memory 320 as calendar data 510. Calendar data 510 may also include user-specific calendar marker data such as user names, reminder notes, preferences, etc.

In practice, the default monitor screen, the main menu screen, or a calendar/clock screen will be displayed when monitor 200 is operating under normal non-alarm conditions. In response to an alarm, however, monitor 200 automatically transitions to an appropriate alarm screen and sounds an audible alert sound if applicable. This particular embodiment of monitor 200 is configured to display three categories of alarms: monitor alarms; pump alarms; and sensor alarms.

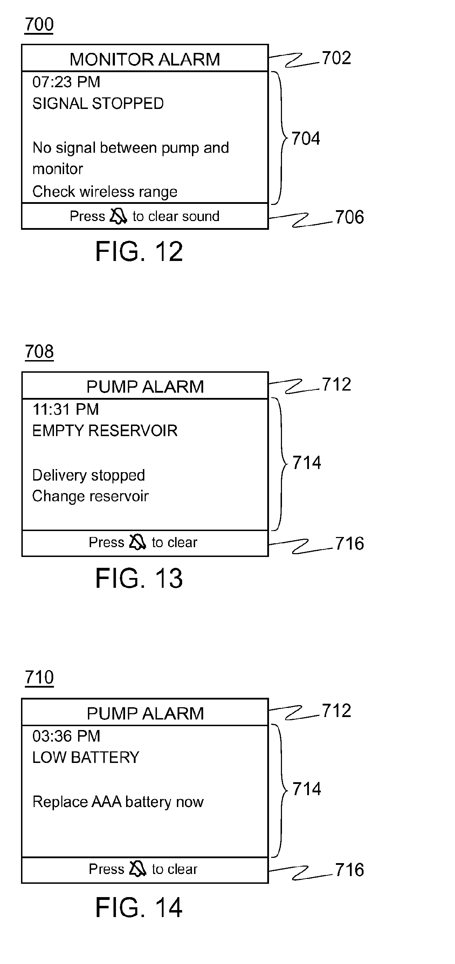

FIG. 12 depicts a monitor alarm screen 700 that may be generated by an embodiment of a monitor. In connection with the display of monitor alarm screen 700, monitor 200 will illuminate alarm snooze button 228 (and possibly night light on/off button 220). As depicted in FIG. 12, monitor alarm screen 700 may include an alarm type field 702, a message field 704, and a user action field 706. Alarm type field 702 indicates the type or category of the alarm, e.g., "Monitor Alarm." The message field 704 is used to display the information related to the current alarm, e.g., the time of the alarm, a brief explanation of the cause or reason for the alarm, and diagnostic instructions (for example, "Check Wireless Range"). Monitor alarm screen 700 may also include user action field 706, which displays user instructions for handling the alarm. The embodiment of monitor 200 described here employs a two-step alarm disable procedure for all alarms and alerts that have both audible and graphical components. First, user action field 706 indicates that alarm snooze button 228 can be pressed to clear the alarm sound. Once the alarm sound is disabled, user action field 706 displays a new message (not shown) that indicates that alarm snooze button 228 can be pressed to clear the alarm message that is currently displayed. Another type of monitor alarm can be generated when the monitor has switched from the normal power source to a backup power source. This backup power alarm may also indicate an estimate of the amount of time remaining on the backup power supply.

The monitor may be suitably configured to accommodate planned absences when the patient (and, therefore, the transmitting source device or devices) will leave the wireless monitoring range of the monitor system. In this regard, monitor 200 may be provisioned with a snooze or disable feature that when activated prevents the generation of "Signal Stopped" or "Out Of Range" alarms. For example, monitor 200 can be set to automatically disable such alarms during known scheduled absences for work or school, where the schedule is programmed into monitor 200 by the user. Alternatively or additionally, these alarms can be manually disabled by the user on an as-needed basis using, e.g., a snooze or disable button. In connection with this feature, the monitor will remain on standby so that it can detect when a transmitting device is again within the wireless range of the system. Upon auto detection of the transmitting device, the "Signal Stopped" or "Out Of Range" alarms are enabled. In practice, the monitor may wait to enable these alarms until after the transmitting device has remained within range for a designated period of time (to contemplate situations where the patient returns for only a brief period before leaving again).

The category of pump alarms includes, without limitation, the following alarms: alarm clock; bolus stopped; E06; low battery; motor error; no delivery; off or no power; auto off; button error; empty reservoir; low reservoir; no reservoir; reset; A23; battery charge warning; check settings; failed battery test; maximum delivery; and weak battery. Of course, any number of electrical, software, hardware, and other alarms can be generated, limited only by the practical implementation considerations. FIG. 13 depicts a pump alarm screen (empty reservoir) 708 that may be generated by an embodiment of a monitor, and FIG. 14 depicts a pump alarm screen (low battery) 710 that may be generated by an embodiment of a monitor. Each pump alarm screen (including those not shown) includes an alarm type field 712, a message field 714, and a user action field 716, each having the general characteristics described above for monitor alarm screen 700. Notably, all pump alarms are disabled by following the two-step alarm disable technique explained above for the monitor alarm.

The monitor generates the alarm clock screen and any associated audio alert in response to an alarm clock notification received from the infusion pump. Message field 714 for the alarm clock screen may include the time of the alarm (and/or the current time) and, if applicable, a brief explanation of the alarm.

The monitor generates the bolus stopped screen and any associated audio alert in response to a respective notification received from the infusion pump. Message field 714 for the bolus stopped screen may include the time of the alarm (and/or the current time) and instructions or questions for the user. For example, message field 714 may display the following text: "Loose battery cap? Pump dropped or bumped? Check bolus history."

The monitor generates the E06 screen and any associated audio alert in response to a respective notification received from the infusion pump. This alarm represents and electrical/software alarm that occurs when the infusion pump has lost one or more configuration settings. Message field 714 for the E06 screen may include the time of the alarm (and/or the current time) and instructions for the user. For example, message field 714 may display the following text: "Settings cleared. Reprogram settings. Call help line for assistance."

The monitor generates low battery screen 710 (FIG. 14) and any associated audio alert in response to a respective notification received from the infusion pump. Message field 714 for low battery screen 710 may include the time of the alarm (and/or the current time) and instructions for the user. For example, message field 714 may display the following text: "Replace AAA battery now." Alternatively, for an infusion pump having a rechargeable battery, message field 714 may display "Recharge battery now."

The monitor generates the motor error screen and any associated audio alert in response to a respective notification received from the infusion pump. This alarm refers to the operation of the infusion pump motor. Message field 714 for the motor error screen may include the time of the alarm (and/or the current time), instructions for the user, and possibly other information. For example, message field 714 may display the following text: "Delivery stopped. Disconnect set."

The monitor generates the no delivery screen and any associated audio alert in response to a respective notification received from the infusion pump. Message field 714 for the no delivery screen may include the time of the alarm (and/or the current time), instructions for the user, and possibly other information. For example, message field 714 may display the following text: "Delivery stopped, change entire set and check glucose. See user guide to troubleshoot."

The monitor generates the off or no power screen and any associated audio alert in response to a respective notification received from the infusion pump. Message field 714 for the off or no power screen may include the time of the alarm (and/or the current time), instructions for the user, status information, etc. For example, message field 714 may display the following text: "No battery life. Delivery stopped. Replace battery now."

The monitor generates the auto off screen and any associated audio alert in response to a respective notification received from the infusion pump. Message field 714 for the auto off screen may include the time of the alarm (and/or the current time) and status information. For example, message field 714 may display the following text: "Delivery stopped. No buttons pushed during time limit."

The monitor generates the button error screen and any associated audio alert in response to a respective notification received from the infusion pump. Message field 714 for the button error screen may include the time of the alarm (and/or the current time) and an explanation for the user. For example, message field 714 may display the following text: "Button pressed for more than three minutes."

The monitor generates empty reservoir screen 708 (FIG. 13) and any associated audio alert in response to a respective notification received from the infusion pump. Message field 714 for empty reservoir screen 708 may include the time of the alarm (and/or the current time), status information, and instructions for the user. For example, message field 714 may display the following text: "Delivery stopped. Change reservoir."

The monitor generates the low reservoir screen and any associated audio alert in response to a respective notification received from the infusion pump. Message field 714 for the low reservoir screen may include the time of the alarm (and/or the current time). In some embodiments, message field 714 need not convey any additional information. In other embodiments, message field 714 may display text or a graphic that indicates how much fluid remains in the reservoir. This indication may represent a volume and/or a time remaining until the reservoir will be empty.

The monitor generates the no reservoir screen and any associated audio alert in response to a respective notification received from the infusion pump. Message field 714 for the no reservoir screen may include the time of the alarm (and/or the current time), status information, and instructions for the user. For example, message field 714 may display the following text: "Delivery stopped. Change reservoir set."

The monitor generates the reset screen and any associated audio alert in response to a respective notification received from the infusion pump. Message field 714 for the reset screen may include the time of the alarm (and/or the current time), a status or an explanation, and instructions for the user. For example, message field 714 may display the following text: "Settings cleared by user. Reprogram settings."

The monitor generates the A23 screen and any associated audio alert in response to a respective notification received from the infusion pump. This alarm occurs when there is a mechanical reset of the infusion pump mechanism. Message field 714 for the A23 screen may include the time of the alarm (and/or the current time), status information, and instructions for the user. For example, message field 714 may display the following text: "Pump reset. Settings preserved."

The monitor generates the battery charge warning screen and any associated audio alert in response to a respective notification received from the infusion pump. This alarm occurs if the pump has been without battery charge for more than a designated period of time, e.g., five minutes. In connection with this alarm, the pump time and date should be checked and updated if necessary. Message field 714 for the battery charge warning screen may include the time of the alarm (and/or the current time) and an explanation or status information for the user. For example, message field 714 may display the following text: "Battery charge, too slow."

The monitor generates the check settings screen and any associated audio alert in response to a respective notification received from the infusion pump. This alarm is generated when it is necessary for the user to check and/or reprogram the infusion pump settings, date, or time. Message field 714 for the check settings screen may include the time of the alarm (and/or the current time), status information, and instructions for the user. For example, message field 714 may display the following text: "Delivery stopped. Reprogram settings."

The monitor generates the failed battery test screen and any associated audio alert in response to a respective notification received from the infusion pump. This alarm occurs if the infusion pump has a used battery that is too low on power to drive the infusion pump. Message field 714 for the failed battery test screen may include the time of the alarm (and/or the current time), status information, and instructions for the user. For example, message field 714 may display the following text: "Delivery stopped. Replace AAA battery now."

The monitor generates the maximum delivery screen and any associated audio alert in response to a respective notification received from the infusion pump. Message field 714 for the maximum delivery screen may include the time of the alarm (and/or the current time), an explanation or status information, and instructions for the user. For example, message field 714 may display the following text: "Exceeded one hour maximum delivery. Check glucose."

The monitor generates the weak battery screen and any associated audio alert in response to a respective notification received from the infusion pump. Message field 714 for the weak battery screen may include the time of the alarm (and/or the current time), an explanation, and instructions for the user. For example, message field 714 may display the following text: "Shorter battery life than expected."

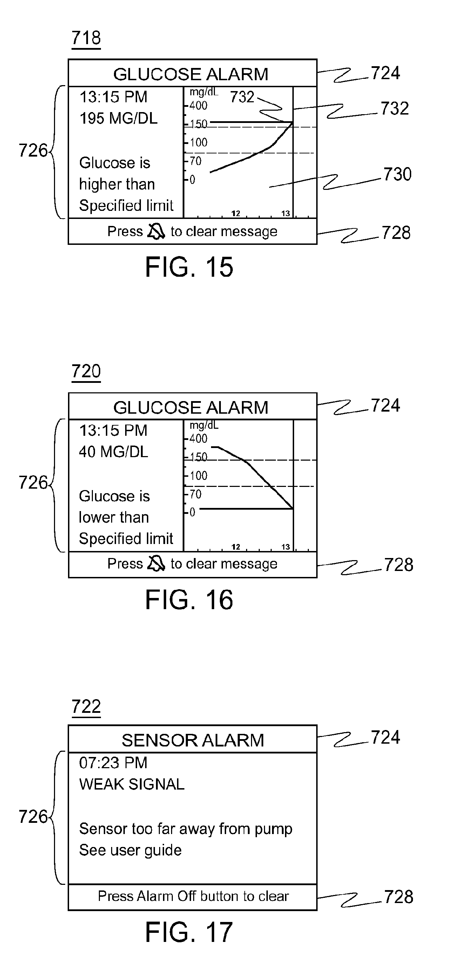

The category of sensor alarms includes, without limitation, the following alarms: high glucose (measured or predicted); low glucose (measured or predicted); glucose rate of change; meter BG reminder; sensor error; weak signal; calibration error; transmitter battery power too low to support normal operation; lost sensor; low transmitter battery power warning; enter meter BG; sensor end; and bad sensor. A glucose level is one example of a monitored physiological characteristic that may be used to trigger an alarm at the monitor. An embodiment of the monitor may be suitably configured to support alarms for other monitored physiological characteristics, and the following description of glucose alarms is not intended to restrict the scope or application of embodiments of the monitor. FIG. 15 depicts a high glucose alarm screen 718, FIG. 16 depicts a low glucose alarm screen 720, and FIG. 17 depicts a sensor alarm (weak signal) screen 722 that may be generated by an embodiment of a monitor. Each glucose alarm screen and each sensor alarm screen (including those not shown) includes an alarm type field 724, a message field 726, and a user action field 728, each having the general characteristics described above for monitor alarm screen 700. Notably, all glucose alarms and sensor alarms are disabled by following the two-step alarm disable technique explained above for the monitor alarm.

The monitor generates high glucose alarm screen 718 and any associated audio alert in response to a respective notification received from the infusion pump and/or the continuous glucose sensor transmitter. In practice, the monitor generates a high glucose alarm if the current status data indicates a high glucose alarm condition. Notably, message field 726 for high glucose alarm screen 718 may include a text message only, a graph only, or a text message displayed with a graph (as depicted in FIG. 15). For high glucose alarm screen 718, the text portion of message field 726 may include the time of the alarm (and/or the current time), a current measurement of the glucose level, and a brief explanation of the alarm. For example, the text portion of message field 726 may display "Glucose is higher than specified limit." As another example, the message may indicate to the user the glucose level that is being exceeded: "Glucose is higher than 240 mg/dL." For high glucose alarm screen 718, the graph portion of message field 726 may include a scaled down version of the full graph, which may be employed by monitor screen 232 (FIG. 8). FIG. 15 depicts a graph 730 of the patient's glucose level (on the vertical scale) versus time (on the horizontal scale). For ease of reading, graph 730 may include lines 732 that correspond to the current glucose level. Moreover, as described above for monitor screen 232, graph 730 may include distinguishable target, hypoglycemic, and hyperglycemic zones. The use of both a text message and a visual graphic may be desirable to ensure that the user can quickly and easily interpret the significance and importance of high glucose alarm screen 718.

A monitor can be used to monitor whether the current status data of the monitored device indicates an emergency condition of a monitored patient. In this regard, certain monitor embodiments may use alarm control logic 412 to generate an emergency glucose level warning if the glucose level exceeds an emergency threshold (such as 400 mg/dL or any user-defined value). For example, if the current glucose level exceeds the threshold, then message field 726 of high glucose alarm screen 718 may include an additional user-defined emergency warning message that is intended to provide important information to a caregiver, first responders, or emergency personnel. The emergency message may read: "Call my spouse at 555-5555" or "Please search for my infant child" or "My medication is in the master bathroom cabinet." Alternatively or additionally, an emergency warning message can be conveyed using graphics, an audio clip, a video clip, or the like.

Furthermore, a monitor can be suitably configured to automatically send a communication to one or more emergency contact persons or entities in response to the detection of certain alarm/alert conditions. In some embodiments, the monitor is configured to establish contact with an emergency contact in response to an urgent alarm/alert and/or in response to the detection of an emergency condition, e.g., low glucose levels. For example, the monitor may automatically dial a specified telephone number (for a doctor, a first-responder, a family member, etc.), automatically send an email to one or more contacts, automatically page a person, or the like. In practice, the emergency contact persons or entities, the manner in which the monitor communicates with the emergency contact persons or entities, the content of the emergency contact messages, alarms, and alerts, and other parameters associated with the emergency contact feature may be user-definable or user-configurable.

The monitor generates low glucose alarm screen 720 and any associated audio alert in response to a respective notification received from the infusion pump and/or the continuous glucose sensor transmitter. The general characteristics and features of low glucose alarm screen 720 are similar to those described above for high glucose alarm screen 718 (notably, however, the text portion of message field 726 may display "Glucose is lower than specified limit" or specify certain levels, for example, "Glucose is lower than 60 mg/dL" or "Glucose is very low: 50 mg/dL").

Certain monitor embodiments may also generate an emergency glucose level warning if the glucose level drops below an emergency threshold (such as 40 mg/dL or any user-defined value). For example, if the current glucose level drops below the threshold, then message field 726 of low glucose alarm screen 720 may include an additional user-defined message that is intended to provide important information to a caregiver, first responders, or emergency personnel. The emergency message may read: "I am diabetic and have gone hypoglycemic" or "Call my doctor at 555-5555" or "I am allergic to peanuts" or "I need orange juice now."

Some monitor embodiments may be configured to request a BG meter reading in response to certain types of alarms (for example, alarms associated with high or low glucose levels). Depending upon the urgency of the alarm, the monitor may instruct the user to obtain a BG meter reading for purposes of accurate calibration before (or soon after) therapy is administered. This feature enables the monitor to collect virtually real time BG meter readings corresponding to critical conditions. The monitor can use such historical data for reporting, calibration, glucose level prediction, etc.

In contrast to glucose alarms, sensor alarms need not (but may) include a glucose graph as described above. For example, weak signal screen 722 represents one such sensor alarm. The monitor generates weak signal screen 722 and any associated audio alert in response to a respective notification received from the infusion pump and/or the continuous glucose sensor transmitter. Message field 726 for weak signal screen 722 may include the time of the alarm (and/or the current time), status information, and instructions for the user. For example, message field 726 may display the following text: "Sensor too far away from pump. See user guide."

The monitor generates the meter BG reminder screen and any associated audio alert in response to a respective notification received from the infusion pump and/or the continuous glucose sensor transmitter. This alarm is intended to remind the user that a BG meter reading needs to be entered soon for purposes of calibration. Message field 726 for the BG reminder screen may include the time of the alarm (and/or the current time), and instructions for the user. For example, message field 726 may display the following text: "This is a reminder to enter meter BG soon."

The monitor generates the sensor error screen and any associated audio alert in response to a respective notification received from the infusion pump and/or the continuous glucose sensor transmitter. This alarm is intended to alert the user of a problem with the sensor. Message field 726 for the sensor error screen may include the time of the alarm (and/or the current time), status information, and instructions for the user. For example, message field 726 may display the following text: "Sensor failed self test. See user guide."

The monitor generates the calibration error screen and any associated audio alert in response to a respective notification received from the infusion pump and/or the continuous glucose sensor transmitter. This alarm is intended to inform the user that a problem occurred in the calibration procedure. Message field 726 for the calibration error screen may include the time of the alarm (and/or the current time), status information or an explanation, and instructions for the user. For example, message field 726 may display the following text: "Invalid sensor data or invalid glucose value. See user guide" or "Try another BG meter reading."

The monitor generates the bad transmitter screen and any associated audio alert in response to a respective notification received from the infusion pump and/or the continuous glucose sensor transmitter. This alarm is intended to inform the user that the continuous glucose sensor transmitter has failed or is performing out of specification. Message field 726 for the bad transmitter screen may include the time of the alarm (and/or the current time), and instructions for the user. For example, message field 726 may display the following text: "Replace transmitter now."

The monitor generates the lost sensor screen and any associated audio alert in response to a respective notification received from the infusion pump and/or the continuous glucose sensor transmitter. This alarm is intended to notify the user when the infusion pump has lost communication with the continuous glucose sensor transmitter. Message field 726 for the lost sensor screen may include the time of the alarm (and/or the current time), an explanation, and instructions for the user. For example, message field 726 may display the following text: "Pump is no longer getting sensor data. See user guide."

The monitor generates the low transmitter screen and any associated audio alert in response to a respective notification received from the infusion pump and/or the continuous glucose sensor transmitter. This alarm is intended to inform the user that the continuous glucose sensor transmitter is transmitting a weak signal or that its battery has become weak. Message field 726 for the low transmitter reminder screen may include the time of the alarm (and/or the current time), and instructions for the user. For example, message field 726 may display the following text: "Replace transmitter now" or "Recharge transmitter when sensor is finished."

The monitor generates the enter meter BG screen and any associated audio alert in response to a respective notification received from the infusion pump and/or the continuous glucose sensor transmitter. This alarm is intended to instruct the user to enter a new BG meter reading. Message field 726 for the enter meter BG screen may include the time of the alarm (and/or the current time), status information, and instructions for the user. For example, message field 726 may display the following text: "Sensor reading invalid. Enter BG value now."

The monitor generates the sensor end screen and any associated audio alert in response to a respective notification received from the infusion pump and/or the continuous glucose sensor transmitter. This alarm is intended to notify the user when the continuous glucose sensor transmitter needs to be replaced. Message field 726 for the sensor end screen may include the time of the alarm (and/or the current time), and instructions for the user. For example, message field 726 may display the following text: "Replace sensor now. See user guide."

The monitor generates the bad sensor screen and any associated audio alert in response to a respective notification received from the infusion pump and/or the continuous glucose sensor transmitter. This alarm is generated when the system detects a problem associated with the continuous glucose sensor transmitter. Message field 726 for the bad sensor screen may include the time of the alarm (and/or the current time), and instructions for the user. For example, message field 726 may display the following text: "Replace sensor now. See user guide."

An embodiment of a monitor may utilize canned messages or content (audio, voice, video, text, etc.) associated with alarms, alerts, or notifications intended for transmission by the monitor. The use of canned pre-programmed messages may be desirable to simplify the operation of the monitor. In this regard, FIG. 6 depicts canned text/voice messages 520 stored in memory 320. Thus, for any of the alarms/alerts described herein, the monitor can retrieve a corresponding message from memory 320 and transmit the message to a designated device (e.g., a mobile telephone, a PDA, a computing device, or a pager). For example, if the monitor generates a low glucose alarm, it can retrieve a canned text message that reads "The patient is hypoglycemic" and send that text as a pager message to the patient's caregiver or any designated contact person. As another example, if the monitor generates a sensor failure or replacement alarm, it can retrieve a canned audio clip such as "Please replace the sensor" and send that audio content as a voicemail message to the patient or any designated contact person.

User-Definable and User-Selectable Features

An embodiment of a monitor may be suitably configured to provide a number of user-selectable, user-configurable, or user-definable settings for items such as alarms, alerts, display settings, operating preferences, and the like. A monitor may include configurable alarm logic 402, configurable display mode logic 404, and other suitably configured processing logic that support the selectable features described herein.

Certain monitor embodiments enable the user to select the types and amount of information to display. This allows the user to customize one or more displays by selecting features, screens, and functions. For example, an embodiment of a monitor gives the user the ability to select and/or control: the timeline for different graphs/plots (e.g., one hour, two hours, and so on, up to 48 hours or any maximum period); the generation of overlapping graphs of different days (where each day's plot can be a different color or line pattern); plots or graphs of insulin deliveries; combined superimposed plots of various data; whether or not BG meter data is displayed; whether or not a numeric glucose display screen is used; which status parameters are displayed; whether or not an alarm history screen is displayed and, if so, the characteristics of the screen; whether or not insulin delivery history is displayed and, if so, the format of the displayed information; whether or not event markers are used and, if so, the specific types.

An embodiment of a monitor may support customizable and/or selectable menu styles, menu structures, themes, languages, or other operational characteristics that accommodate the particular needs of different users. For example, the monitor may include a theme that is designed to appeal to children; such a theme may utilize easy-to-understand instructions, bright and simple display features, tutorials presented by likeable cartoon characters, etc. As another example, the monitor may be configurable to accommodate those who are hard of hearing (e.g., reduced dependence on audio instructions, increased volume, increased use of non-audible alarms and alerts). The monitor may also be configurable to accommodate those with poor eyesight (e.g., reduced dependence on displayed instructions, larger display fonts, increased use of audible alarms and alerts).

The monitor may be configured to generate audible alerts that are identical or similar to those generated by the infusion pump itself. This enables the caregiver/patient to easily recognize the alerts generated by the monitor, assuming that he or she is already familiar with the alerts generated by the infusion pump. In other words, the caregiver/patient need not learn the meaning and context of new alert tones and patterns. Alternatively (or additionally), an embodiment of a monitor may employ configurable alarm logic 402 that supports user-selectable, user-definable, and/or customizable alarms and alerts for one or more of the different alarms generated by the monitor. For example, the monitor may allow the user to select different types of alarms, such as a vibration alarm, an audible alarm, flashing lights (which may be realized in the main display element, in the front or top panel, in the night light, etc.), displayed images, or the like. As another example, the monitor may allow the user to select one or more characteristics associated with an alarm, such as: the volume (for audible alarms); an escalating volume characteristic; the duration of the alarm; the magnitude of a vibrating alarm; the brightness of a displayed alarm; the specific tone, tone pattern, recorded audio or voice messages, or music played for an audible alarm; the audio, text, or video content contained in a transmittable alarm or alert message; or the like. Moreover, the monitor may allow the user to define and select the alarm characteristics according to the user's schedule or status. For example, different alarm types and/or different alarm characteristics may be utilized for the following (and other) scenarios: when the user is asleep; when the user is watching television; when the user is on the telephone; when the user is exercising; when the user is eating; or the like. Thus, the user can customize the alarms as needed to prioritize the alarms relative to the current living conditions and state of affairs. In practice, these user-configurable settings may be accessible via a utilities or preferences submenu of the monitor, and the settings can be stored as user preferences data 508 in memory 320 (FIG. 6).

An embodiment of a monitor may support voice recorded alarm/alert messages. Referring to FIG. 4, microphone 313 can be utilized to accommodate the recording of customized voice or audio clips. The customized voice messages can be played by the monitor and/or transmitted by the monitor as alarm messages. This feature can be desirable for certain users who respond better to recognizable voices (rather than an unfamiliar voice or a computer-generated voice). For example, a patient will usually pay attention to an alarm message that contains a stem warning in the voice of a parent or a doctor.

An embodiment of a monitor may include configurable display mode logic 404 that supports user-selectable, user-definable, and/or customizable display settings or display modes for the monitor. For example, the monitor may allow the user to select different display characteristics, including, without limitation: a continuous display mode; a periodic display mode where a monitor screen or a home screen appears at scheduled times; an alarm-triggered display mode that only displays information when the glucose level is above/below a specified threshold level; a manual display mode that only displays information at the command of the user; or the like. Referring again to FIG. 8 and FIG. 9 and the associated description, monitor 200 may be suitably configured to generate its various display screens in response to user-configurable display settings. As another example, the monitor may allow the user to select one or more display or screen characteristics, such as the overall screen brightness, the duration of a displayed screen, the font size of displayed text messages, the colors associated with a displayed screen and/or individual graphical elements of a screen, the graphical content of a displayed screen, or the like. Moreover, the monitor may allow the user to define and select the various display characteristics according to the user's schedule or status. For example, different display types, display modes, and/or display features may be utilized for the following (and other) scenarios: when the user is asleep; when the user is watching television; when the user is on the telephone; when the user is exercising; when the user is eating; or the like. Thus, the user can customize the monitor display as needed to prioritize alarms and/or to suit the current living conditions and state of affairs. In practice, these user-configurable settings may be accessible via a utilities or preferences submenu of the monitor, and the settings can be stored as user preferences data 508 in memory 320 (FIG. 6).

The monitor may also be configured to allow the user to select the recipients for certain alarm and alert messages. For instance, the user can manipulate the user interface of the monitor to enter contact information (such as a name, a telephone number, an email address, a fax number, or a pager number) for a person or an entity. In addition, the monitor may allow the user to link one or more persons or entities to any given alarm or alert. This feature allows the user to configure the messaging functionality of the monitor. Thus, the user can configure the monitor such that routine alerts are routed to the user only, while urgent alarms are routed to the user, a family member, and a first responder.

An embodiment of a monitor may be configured to process event markers, which indicate the occurrence of an event that might be relevant to the user. For example, event markers may be utilized to identify when the user is eating or exercising. Depending upon its context, an event marker may have an associated start time, end time, duration, and/or magnitude (that represents an anticipated adjustment in a physiological characteristic such as glucose level). Event markers may be processed in connection with the generation of a glucose graph for the user (see FIG. 8, FIG. 15, and FIG. 16). In this regard, the glucose plot of a graph may include indicia that represents the beginning and/or the end of the particular event. The indicia may also convey information related to the type, context, or definition of the event. For example, an "E" may be used as an event marker for a period of eating or exercise, while an "S" may be used as an event marker for a period of sleep. The event markers may alternatively (or additionally) be realized using different colors, brightness, text, patterns, or any visually distinguishable characteristics.

A monitor may be preprogrammed with a number of common event markers. In certain embodiments, the monitor is suitably configured to support selectable, user-definable, or customizable event markers for use with the current status data of the monitored medical device. In practice, such user-defined event markers may be entered at the user interface of the monitor itself, programmed via an external computing device, transferred to the monitor via a portable memory device, or the like. Accordingly, the monitor allows the user to create his or her own event markers as needed without being restricted to a limited number of canned event markers. Such user-defined markers may include, without limitation: a specific form of exercise, e.g., running, swimming, skateboarding, yoga; the beginning of a menstrual cycle; taking a dose of specified medication; feeling work stress; feeling tired due to insufficient sleep; drinking alcohol; eating specific types of food; illness; etc. Moreover, the monitor may allow the user to select different display characteristics associated with event markers, including, without limitation: alphanumeric characters; colors; icons, graphics, or other content; displayed marker size; brightness; the font size of displayed alphanumeric markers; or the like. This feature allows the user to personalize and customize the monitor as needed to accommodate his or her lifestyle and schedule. In practice, the monitor can save the settings for these user-defined event markers 518 in memory 320 (FIG. 6).

Alarm and Alert Features

This section describes a number of techniques, technologies, and features related to the manner in which an embodiment of a monitor generates and handles alarms and alerts. In practice, some of these techniques, technologies, and features may be optional.

For this exemplary embodiment, the monitor itself generates monitor alarms in response to self diagnostic processes. On the other hand, the monitor generates pump and sensor alarms in response to data received from the infusion pump and/or data received from the continuous glucose sensor transmitter. In one embodiment, the infusion pump functions as a relay device for any sensor alarms that might originate at the continuous glucose sensor transmitter. For such an embodiment, pump data transmitted by the infusion pump triggers the various pump and sensor alarms at the monitor.

The monitor may be suitably configured to automatically adjust its volume (for alarms, alerts, tutorials, or any audio-visual item generated by the monitor) in response to the ambient or environmental noise characteristics. For example, microphone 313 (FIG. 4) can be utilized to sample or otherwise receive a sound level associated with the ambient noise near the monitor. In response, the monitor can adjust its volume upwardly or downwardly as needed. For example, the monitor may increase its volume under noisy conditions such that alarms and alerts will be heard over the ambient noise. In contrast, the monitor may decrease its volume under quiet conditions such that alarms and alerts are not generated at an overpowering level.

In practice, an alarm can be active at both the infusion pump (or sensor transmitter) and the monitor. An embodiment of a monitor may be configured such that the infusion pump functions as a master device for purposes of alarm management. In such an embodiment, snoozing, clearing, or disabling an alarm at the monitor will have no effect on the redundant alarm being generated by the infusion pump. Thus, the user will still need to clear the alarm at the infusion pump itself (this forces the user to actually check the status of the infusion pump). If the user does not clear the alarm at the infusion pump, then the monitor may receive a subsequent alarm message from the infusion pump (corresponding to the same alarm), which will again trigger the alarm at the monitor. On the other hand, clearing an alarm at the infusion pump will initiate the cancellation of the redundant alarm at the monitor (for example, the infusion pump will transmit a "clear alarm" message to the monitor for that particular alarm). An alternate embodiment of a monitor may treat both the infusion pump and the monitor as master devices for purposes of alarm management.

As described above with reference to FIG. 11, the monitor may be capable of storing alarm history data. This feature may be desirable to ensure that the user does not miss any alarms that may have been generated during periods of absence or during periods when the monitor has been silenced. The monitor may also be suitably configured to prioritize the handling of multiple alarms, whether or not such alarms are saved in history. For example, a low glucose alarm may be treated as a top priority item that is always generated first if multiple alarms are active. As another example, the monitor may queue alarms such that, in response to the silencing of a currently active alarm, the next alarm is immediately generated. In one embodiment, the monitor functions as a slave device to the infusion pump, which functions as the master device for purposes of alarm prioritization. In another embodiment, the monitor itself may include processing logic that prioritizes alarms and alerts. As mentioned above, such prioritization and other alarm settings may be user-configurable.

Referring again to FIG. 8, a monitor as described herein is capable of displaying monitor screen 232 having a glucose graph that depicts the patient's glucose level over time. Moreover, referring to FIG. 15 and FIG. 16, a monitor as described herein is capable of generating high glucose alarm screen 718 or low glucose alarm screen 720 when the patient's glucose level is outside of the designated target (safe) zone. An embodiment of the monitor may also be suitably configured to generate a glucose graph that extrapolates the patient's historical or empirical glucose data and predicts how the patient's glucose level will trend in the near future. In this regard, the monitor may employ an appropriate predictive glucose algorithm 414 (FIG. 5) or any suitably configured processing architecture, processing logic, or component that analyzes sensor data obtained by the monitor, where that sensor data indicates empirical glucose measurements of a monitored patient. Processing the received sensor data facilitates estimation of future glucose measurements based upon the empirical glucose measurements, event markers, and possibly other information or data available to the monitor.

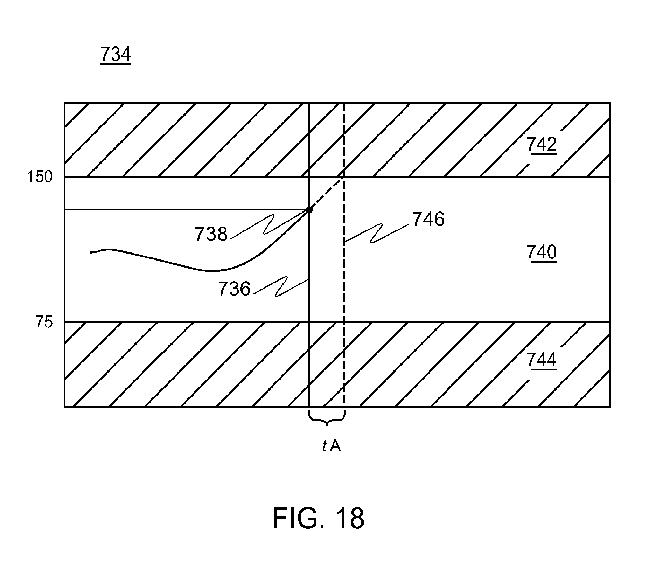

FIG. 18 depicts one example of a predictive glucose graph 734 that may be generated by a monitor in connection with the display and rendering of a monitor screen, an alarm screen, a home screen, or the like. As with the graph depicted in FIG. 8, the vertical scale of graph 734 represents the glucose level in mg/dL, and the horizontal scale of graph 734 represents time. The vertical line 736 indicates the current time. Thus, the intersection point 738 of vertical line 736 and the plot represents the currently measured glucose level. Graph 734 may include indicia of a desired target zone 740, a hyperglycemic zone 742, and a hypoglycemic zone 744 as described above for monitor screen 232. Notably, the solid portion of the plot corresponds to historical and actual glucose data, while the dashed portion of the plot corresponds to predicted or anticipated glucose levels that are calculated based upon historical glucose trends. In this regard, predictive glucose graph 734 functions to graphically indicate the predicted future glucose measurements.

The monitor may utilize extrapolation techniques, curve fitting techniques, and/or other predictive or estimation techniques and technologies to process the empirical glucose measurement data and to estimate the future glucose measurements. Thus, the future glucose measurements may be based upon extrapolated data and/or curve fitted data. For this example, the predicted portion of the plot generally follows the rising slope and increasing glucose trend associated with the actual measured glucose levels. The monitor may be configured to predict glucose levels for a designated window of time in the future (for example, thirty minutes), and that window of time may be dictated by the accuracy of the predictive algorithm.

Notably, the monitor can be designed to generate an alarm and/or display a warning screen if it determines that the patient's glucose level will leave target zone 740 in the near future. In this regard, certain monitor embodiments may use alarm control logic 412 to generate a warning alarm when predicted future glucose measurements indicate an alarm condition for the patient's glucose level. In other words, the monitor can alert the user before the glucose level actually leaves target zone 740. In practice, predictive glucose algorithm 414 may be configured to predict whether the future glucose measurements will leave target zone 740 within a predetermined period of time, e.g., a thirty minute window. Moreover, the monitor may be suitably configured to obtain an estimated time corresponding to when the patient's future glucose level will leave target zone 740. This estimated time is labeled *t_{A}* in FIG. 18. In practice, predictive glucose graph 734 may include an indicator of the estimated time, e.g., the period of time corresponding to *t_{A}*, the predicted time when the future glucose level will leave target zone 740, or the like. FIG. 18 depicts a scenario where the patient's glucose level is predicted to exceed the designated upper glucose threshold after the time period of *t_{A}*, and the dashed vertical line 746 represents the predicted time when the glucose level will leave target zone 740. The monitor may also be configured to generate, maintain, and display a countdown timer corresponding to the estimated time.

An embodiment of a monitor may include a temporary alarm disable feature (which may be realized or performed by temporary alarm disabling logic 416) that allows the monitor to temporarily disable an alarm function when the current status data indicates an anticipated change in the monitored glucose level, where that anticipated change would otherwise trigger an alarm. In certain embodiments, this feature cooperates with a carbohydrate or bolus estimator that estimates a bolus based on carbohydrate consumption, assists the user with carbohydrate counting, and assists the user in determining precise dosing adjustments to account for meals. The bolus estimator may be implemented in the monitor itself and/or in the infusion pump. United States patent number 7,109,878 describes a bolus estimator in detail; the relevant portions of this patent are incorporated herein by reference.

In practice, the bolus estimator considers expected carbohydrate intake to calculate a recommended insulin bolus that will compensate for the increased glucose level. Under some conditions, the patient's glucose level may briefly exceed his or her target level even though the recommended bolus has been administered. A monitor as described here can be suitably configured to process information generated by the bolus estimator (or otherwise related to the operation of the bolus estimator), anticipate temporary departures from the patient's target zone, and temporarily disable high/low glucose alarms to reduce the likelihood of nuisance alarms. In practice, this effectively expands the range of the patient's target zone for a short time period following the delivery of the recommended bolus. After a desired settling time, the nominal target zone of the patient will be reinstated and/or the glucose alarms will be enabled.

Exemplary Operating Methods

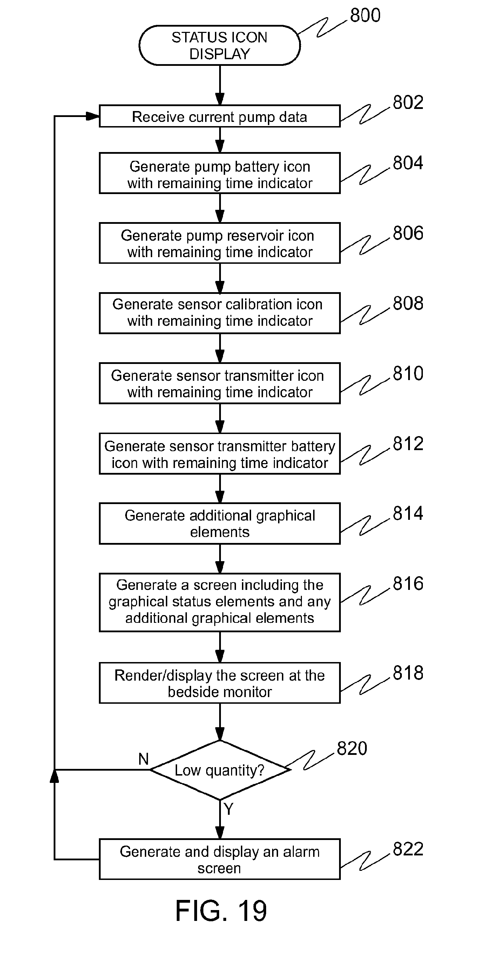

FIG. 19 is a flow chart that illustrates an embodiment of a status icon display process 800 for a monitor. The various tasks performed in connection with process 800 may be performed by software, hardware, firmware, or any combination thereof. For illustrative purposes, the following description of process 800 may refer to elements mentioned above in connection with FIGS. 1-18. In practice, portions of process 800 may be performed by different elements of the described system. It should be appreciated that process 800 may include any number of additional or alternative tasks, the tasks shown in FIG. 19 need not be performed in the illustrated order, and process 800 may be incorporated into a more comprehensive procedure or process having additional functionality not described in detail herein.

For this example, status icon display process 800 is described in the context of a monitor communicating with an infusion pump. Process 800 may begin with the monitor receiving current status data (e.g., pump data) from the infusion pump (task 802). As mentioned above, the pump data may include current status data of the infusion pump itself and current status data of a continuous glucose sensor transmitter, which communicates with the infusion pump. The monitor can process the received pump data in the manner described in the above sections. In response to the current pump data, the monitor generates at least one status element that graphically indicates a remaining measurement (relative to time) for an exhaustible operating quantity of the infusion pump or the continuous glucose sensor transmitter.

For example, referring again to FIG. 8, status icon display process 800 may generate a pump battery icon with a corresponding alphanumeric remaining time indicator (task 804). Alternatively or additionally, process 800 may generate a pump reservoir icon and a corresponding alphanumeric remaining time (or volume) indicator (task 806). Alternatively or additionally, process 800 may generate a sensor calibration icon and a corresponding alphanumeric remaining time indicator (task 808). Alternatively or additionally, process 800 may generate a sensor transmitter icon and a corresponding alphanumeric remaining time indicator (task 810). Alternatively or additionally, process 800 may generate a sensor transmitter battery icon and a corresponding alphanumeric remaining time indicator (task 812). Alternatively or additionally, process 800 may generate any number of additional graphical elements (task 814), including any of the graphical features, elements, icons, or components described above.

Next, the monitor generates a screen (e.g., a monitor screen, an alarm screen, a menu screen, a home screen, etc.) that includes one or more of the graphical status elements and any additional graphical elements as needed (task 816). This screen can then be rendered and displayed on the display element of the monitor (task 818). FIG. 8 depicts an exemplary monitor screen 232 that includes the five graphical status elements mentioned above. In certain embodiments, the monitor may analyze the current status data to determine whether the current status data indicates that an exhaustible operating quantity has reached a low threshold value (query task 820). If not, then status icon display process 800 may be re-entered at task 802 for purposes of updating. If, however, query task 820 detects an alarm condition, then the monitor may generate and display an appropriate alarm screen (task 822). As one example, FIG. 14 depicts a pump alarm screen 710 that indicates a low pump battery condition. After the alarm screen has been cleared, process 800 may be re-entered at task 802.

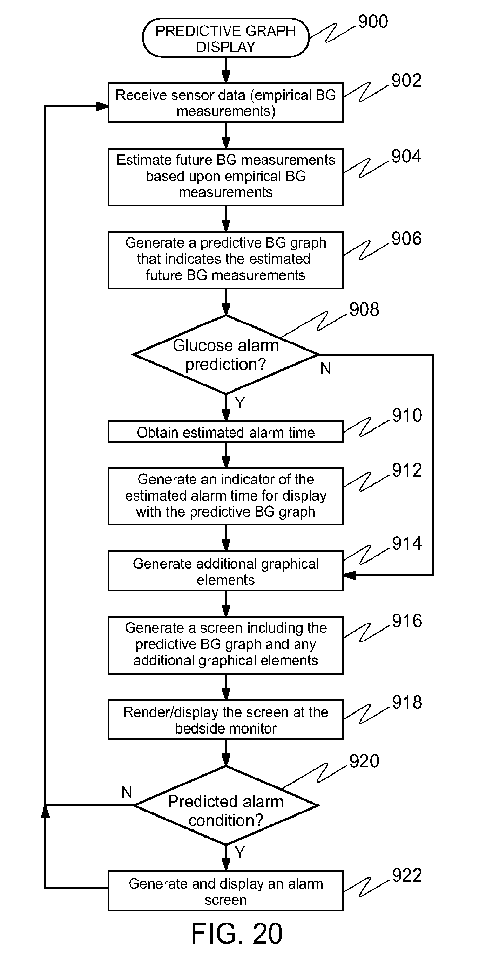

FIG. 20 is a flow chart that illustrates an embodiment of a predictive graph display process 900 for a monitor. The various tasks performed in connection with process 900 may be performed by software, hardware, firmware, or any combination thereof. For illustrative purposes, the following description of process 900 may refer to elements mentioned above in connection with FIGS. 1-18. In practice, portions of process 900 may be performed by different elements of the described system. It should be appreciated that process 900 may include any number of additional or alternative tasks, the tasks shown in FIG. 20 need not be performed in the illustrated order, and process 900 may be incorporated into a more comprehensive procedure or process having additional functionality not described in detail herein.

For this example, predictive graph display process 900 is described in the context of a monitor that receives and processes glucose data that originates from a continuous glucose sensor transmitter. Alternate embodiments of process 900 can be employed to handle other types of physiological characteristic data. Process 900 may begin with the monitor receiving sensor data (e.g., pump data) that indicates empirical measurements of the glucose level of a monitored patient (task 902). As mentioned above, the pump data may include current status data of the infusion pump itself and current status data of a continuous glucose sensor transmitter, which communicates with the infusion pump. The monitor can process the received sensor data in the manner described in the above sections. In particular, the monitor estimates future measurements of the patient's glucose level, based upon the collected sensor data (task 904). As mentioned above, an embodiment of a monitor may extrapolate the empirical glucose measurements to obtain extrapolated data, where the future glucose measurements are based upon or derived from the extrapolated data. Alternatively or additionally, an embodiment of a monitor may perform curve fitting on the empirical glucose measurements to obtain curve fitted data, where the future glucose measurements are based upon or derived from the curve fitted data. For this embodiment, the monitor generates a predictive glucose graph that graphically indicates the estimated future glucose measurements (task 906). An example of such a predictive glucose graph is depicted in FIG. 18, and certain characteristics and features of a predictive glucose graph were described above with reference to FIG. 18.

For this example, predictive graph display process 900 predicts whether the future glucose measurements will leave the patient's target glucose zone within a designated period of time in the future. In this regard, if process 900 predicts that a glucose alarm will be necessary (query task 908), then it may proceed to obtain an estimated time corresponding to when the future glucose measurement will leave the target zone (task 910). In addition, process 900 may generate an indicator of the estimated time for display with the predictive glucose graph (task 912). Alternatively or additionally, process 900 may generate any number of additional graphical elements (task 914), including any of the graphical features, elements, icons, or components described above. Process 900 may also lead to task 914 if the monitor does not predict a future glucose alarm (query task 908).

Next, the monitor generates a screen (e.g., a monitor screen, an alarm screen, a menu screen, a home screen, etc.) that includes the predictive glucose graph, the estimated time indicator, and any additional graphical elements as needed (task 916). This screen can then be rendered and displayed on the display element of the monitor (task 918). In certain embodiments, the monitor may analyze the sensor data and/or the estimated future glucose measurements to determine whether the future glucose measurements indicate an alarm condition for the patient's glucose level. Thus, if predictive graph display process 900 predicts an alarm condition (query task 920), then the monitor may generate and display an appropriate alarm screen (task 922). Such an alarm screen may serve as a warning to the user to expect a glucose alarm in the near future. After the alarm screen has been cleared, process 900 may be re-entered at task 902 for purposes of updating. Similarly, if query task 920 does not predict an alarm condition, process 900 may be re-entered at task 902.

While at least one example embodiment has been presented in the foregoing detailed description, it should be appreciated that a vast number of variations exist. It should also be appreciated that the example embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application.
Further embodiments are set out in the paragraphs that follow:
Paragraph 1. A monitor device (200) for a system comprising a medical device having an exhaustible operating quantity, the monitor device (200) comprising: a display element (208); a display controller/driver (418) coupled to the display element (208) and configured to generate a screen for rendering on the display element (208); and a data communication module (302, 304) configured to receive current status data of the medical device, the current status data indicating a status of the exhaustible operating quantity; wherein the screen comprises a status icon that graphically indicates the status of the exhaustible operating quantity.
Paragraph 2. A monitor device (200) according to paragraph 1, wherein: the medical device comprises an infusion pump (104) having a battery; the exhaustible operating quantity is a charge of the battery; and the status is a remaining charge time for the battery.
Paragraph 3. A monitor device (200) according to paragraph 1, wherein: the medical device comprises an infusion pump (104) having a reservoir; the exhaustible operating quantity is an amount of fluid in the reservoir; and the status is a remaining time until the reservoir will be empty.
Paragraph 4. A monitor device (200) according to paragraph 1, wherein: the medical device comprises an infusion pump (104) having a reservoir; the exhaustible operating quantity is an amount of fluid in the reservoir; and the status is a remaining volume of fluid in the reservoir.
Paragraph 5. A monitor device (200) according to paragraph 1, wherein: the medical device comprises a physiological characteristic sensor transmitter (106) having a battery; the exhaustible operating quantity is a charge of the battery; and the status is a remaining charge time for the battery.
Paragraph 6. A monitor device (200) according to paragraph 1, wherein: the medical device comprises a physiological characteristic sensor (106); the exhaustible operating quantity is a calibration period associated with the physiological characteristic sensor (106); and the status is a remaining time until the calibration period ends.
Paragraph 7. A monitor device (200) according to paragraph 1, wherein: the medical device comprises a physiological characteristic sensor (106); the exhaustible operating quantity is a replacement period associated with the physiological characteristic sensor (106); and the status is a remaining time until the replacement period ends.
Paragraph 8. A monitor device (200) according to paragraph 1, wherein the data communication module (302, 304) is configured to receive the current status data from the medical device.
Paragraph 9. A monitor device (200) according to paragraph 8, wherein the data communication module (302, 304) is a wireless data communication module (302) configured to receive the current status data from the medical device via a wireless link.
Paragraph 10. A monitor device (200) according to paragraph 1, wherein the system is an insulin infusion system (100) and the medical device is an insulin pump (104).
Paragraph 11. A monitor device (200) according to paragraph 1, further comprising: alarm control logic (412) configured to generate alarms associated with the operation of the medical device; and configurable alarm logic (402) that influences the alarm control logic (412), the configurable alarm logic (402) supporting user-selectable and user-definable alarms for the monitor device (200).
Paragraph 12. A monitor device (200) according to paragraph 1, further comprising configurable display mode logic (404) coupled to the display controller / driver (418), the configurable display mode logic (404) supporting user-selectable and user-definable display characteristics for the monitor device (200).
Paragraph 13. A monitor device (200) according to paragraph 1, further comprising a memory element (320) configured to store at least one user-defined event marker (518) for use with the current status data.
Paragraph 14. A monitor device (200) according to paragraph 1, further comprising alarm control logic (412) configured to generate a user-defined emergency warning message when the current status data indicates an emergency condition of a monitored patient.
Paragraph 15. A monitor device (200) according to paragraph 1, further comprising a memory element (320) configured to store user-defined personalization data (506), wherein the display controller/driver (418) is configured to generate a graphical representation of the user-defined personalization data (506) for rendering on the display element (208).
Paragraph 16. A monitor device (200) according to paragraph 15, wherein the user-defined personalization data (506) is selected from the group consisting of image data, avatar data, user initials data, user name data, digital photograph data, graphics data, and alphanumeric data.
Paragraph 17. A monitor device (200) according to paragraph 1, further comprising alarm control logic (412) configured to generate a physiological characteristic alarm when the current status data indicates an alarm condition for a monitored physiological characteristic, wherein the display controller/driver (418) is configured to generate an alarm screen (718, 720) corresponding to the physiological characteristic alarm, the alarm screen (718, 720) comprising a text message displayed with a graph of the monitored physiological characteristic.
Paragraph 18. A monitor device (200) according to paragraph 17, wherein the text message comprises a current measurement of the monitored physiological characteristic.
Paragraph 19. A monitor device (200) according to paragraph 1, further comprising a memory element (320) configured to store alarm history data, wherein the display controller / driver (418) is configured to generate an alarm history screen (274) comprising a selectable list of alarms corresponding to the alarm history data.
Paragraph 20. A monitor device (200) according to paragraph 1, further comprising temporary alarm disabling logic (416) configured to temporarily disable an alarm function of the monitor device (200) when the current status data indicates an anticipated change in a monitored physiological characteristic, where the anticipated change would otherwise trigger an alarm.
Paragraph 21. A monitor device (200) according to paragraph 1, wherein the monitor device (200) configures the data it displays depending upon a device type of the medical device.
Paragraph 22. A monitor device (200) according to paragraph 1, wherein the monitor device (200) configures the data it displays depending upon a quantity of supported medical devices.
Paragraph 23. A monitor device (200) according to paragraph 1, further comprising means for causing an alarm to snooze using a single touch.
Paragraph 24. A monitor device (200) according to paragraph 1, further comprising memory (320) for storing a sleeping pattern of a patient.
Paragraph 25. A monitor device (200) according to paragraph 24, wherein the monitor device (200) is configured to support at least one alarm having first characteristics for when the patient is sleeping and second characteristics for when the patient is awake.
Paragraph 26. A monitor device (200) according to paragraph 24, further comprising means for generating an alert before a sleep time of the patient if at least one exhaustible quantity will become depleted or expire before an awake time of the patient.
Paragraph 27. A monitor device (200) according to paragraph 1, further comprising a temporary alarm disabling feature (416) that when activated prevents generation of an out-of- wire less- range alarm.
Paragraph 28. A monitor device (200) according to paragraph 27, further comprising means for automatically detecting when the medical device is in communication range to re-enable disabled alarms.
Paragraph 29. A monitor device (200) according to paragraph 28, wherein the monitor device (200) re-enables disabled alarms only after the medical device is within communication range for a predetermined period of time.
Paragraph 30. A monitor device (200) according to paragraph 1, further comprising a setup wizard.
Paragraph 31. A monitor device (200) according to paragraph 1, further comprising tutorial clips.
Paragraph 32. A monitor device (200) according to paragraph 31, wherein when an alarm is generated the monitor device (200) displays an option for a user to select a tutorial clip related to the alarm.
Paragraph 33. A monitor device (200) according to paragraph 1, further comprising a plurality of user-selectable display themes.
Paragraph 34. A monitor device (200) according to paragraph 1, further comprising user defined alarm characteristics according to a schedule or status of a user.
Paragraph 35. A monitor device (200) according to paragraph 1, further comprising means to prioritize multiple alarms.
Paragraph 36. A monitor device (200) according to paragraph 1, further comprising means to queue multiple alarms.
Paragraph 37. A method (800) of operating a monitor device (200) for a system (100) comprising a medical device having an exhaustible operating quantity, the method comprising: receiving (802) current status data of the medical device, the current status data indicating a remaining measurement for the exhaustible operating quantity; generating a status element that graphically indicates the remaining measurement relative to time; and displaying (818) the status element at the monitor device.
Paragraph 38. A method (800) according to paragraph 37, wherein displaying (818) the status element comprises rendering a monitor screen for the monitor device (200), the monitor screen including the status element.
Paragraph 39. A method (800) according to paragraph 37, wherein: the medical device comprises an infusion pump (104) having a battery; the exhaustible operating quantity is a charge of the battery; the remaining measurement is a remaining charge time for the battery; and the status element comprises a battery icon and an alphanumeric time indicator.
Paragraph 40. A method (800) according to paragraph 37, wherein: the medical device comprises an infusion pump (104) having a reservoir; the exhaustible operating quantity is an amount of fluid in the reservoir; the remaining measurement is a remaining time until the reservoir will be empty; and the status element comprises a reservoir icon and an alphanumeric time indicator.
Paragraph 41. A method (800) according to paragraph 37, wherein: the medical device comprises an infusion pump (104) having a reservoir; the exhaustible operating quantity is an amount of fluid in the reservoir; the remaining measurement is a remaining volume of fluid in the reservoir; and the status element comprises a reservoir icon and an alphanumeric volume indicator.
Paragraph 42. A method (800) according to paragraph 37, wherein: the medical device comprises a physiological characteristic sensor transmitter (106) having a battery; the exhaustible operating quantity is a charge of the battery; the remaining measurement is a remaining charge time for the battery; and the status element comprises a battery icon and an alphanumeric time indicator.
Paragraph 43. A method (800) according to paragraph 37, wherein: the medical device comprises a physiological characteristic sensor (106); the exhaustible operating quantity is a calibration period associated with the physiological characteristic sensor (106); the remaining measurement is a remaining time until the calibration period ends; and the status element comprises a calibration icon and an alphanumeric time indicator.
Paragraph 44. A method (800) according to paragraph 37, wherein: the medical device comprises a physiological characteristic sensor (106); the exhaustible operating quantity is a replacement period associated with the physiological characteristic sensor (106); the remaining measurement is a remaining time until the replacement period ends; and the status element comprises a sensor transmitter icon and an alphanumeric time indicator.
Paragraph 45. A method (800) according to paragraph 37, wherein receiving (802) the current status data comprises wirelessly receiving the current status data from the medical device.
Paragraph 46. A method (800) according to paragraph 37, further comprising generating (822) an alarm screen at the monitor device if the current status data indicates that the exhaustible operating quantity has reached a low threshold value.
Paragraph 47. A method (800) according to paragraph 37, further comprising: maintaining a sleeping pattern for a user of the medical device, the sleeping pattern indicating a normal bedtime and a normal waking time of the user; and generating an alert before the normal bedtime if the monitor device predicts that the exhaustible operating quantity will become depleted before the normal waking time.
Paragraph 48. A monitor device (200) for a medical device system (100), the monitor device (200) comprising: a display element (208); a display controller/driver (418) coupled to the display element (208) and configured to generate a screen for rendering on the display element (208); and a processing architecture (318) configured to: analyze sensor data obtained by the monitor device (200), the sensor data indicating empirical measurements of a physiological characteristic of a monitored patient; and estimate future measurements of the physiological characteristic based upon the sensor data; wherein the screen comprises a predictive graph (734) of the physiological characteristic that graphically indicates the empirical measurements and the future measurements.
Paragraph 49. A monitor device (200) according to paragraph 48, wherein the predictive graph (734) comprises indicia of a target zone (740) for the physiological characteristic.
Paragraph 50. A monitor device (200) according to paragraph 49, the processing architecture (318) being configured to predict whether the future measurements will leave the target zone (740) within a period of time.
Paragraph 51. A monitor device (200) according to paragraph 50, wherein: the processing architecture (318) is configured to obtain an estimated time (746) corresponding to when the future measurements will leave the target zone (740); and the screen comprises an indicator of the estimated time.
Paragraph 52. A monitor device (200) according to paragraph 51, wherein the indicator comprises a countdown timer.
Paragraph 53. A monitor device (200) according to paragraph 48, further comprising a wireless data communication module (302) configured to wirelessly receive the sensor data from an infusion pump (104).
Paragraph 54. A monitor device (200) according to paragraph 48, further comprising a wireless data communication module (302) configured to wirelessly receive the sensor data from a continuous physiological sensor transmitter (106).
Paragraph 55. A monitor device (200) according to paragraph 48, wherein: the medical device system (100) is an insulin infusion system having a continuous glucose sensor transmitter (106); and the sensor data comprises blood glucose data.
Paragraph 56. A monitor device (200) according to paragraph 48, further comprising alarm control logic (412) configured to generate a warning alarm when the future measurements indicate an alarm condition for the physiological characteristic of the monitored patient, wherein the display controller/driver (418) is configured to generate an alarm screen corresponding to the warning alarm.
Paragraph 57. A monitor device (200) according to paragraph 48, the processing architecture (318) being configured to extrapolate the empirical measurements, resulting in extrapolated data, wherein the future measurements are based upon the extrapolated data.
Paragraph 58. A monitor device (200) according to paragraph 48, the processing architecture (318) being configured to perform curve fitting on the empirical measurements, resulting in curve fitted data, wherein the future measurements are based upon the curve fitted data.
Paragraph 59. A method (900) of operating a monitor device (200) for a medical device system (100), the method (900) comprising: receiving (902) sensor data that indicates empirical measurements of a physiological characteristic of a monitored patient; estimating (904) future measurements of the physiological characteristic based upon the sensor data; generating (906) a predictive graph of the physiological characteristic, where the predictive graph graphically indicates the empirical measurements and the future measurements; and displaying (918) the predictive graph at the monitor device.
Paragraph 60. A method (900) according to paragraph 59, wherein displaying (918) the predictive graph comprises rendering a monitor screen for the monitor device, the monitor screen including the predictive graph.
Paragraph 61. A method (900) according to paragraph 59, wherein receiving (902) the sensor data comprises wirelessly receiving the sensor data from an infusion pump (104).
Paragraph 62. A method (900) according to paragraph 59, wherein receiving (902) the sensor data comprises wirelessly receiving the sensor data from a continuous glucose sensor transmitter (106).
Paragraph 63. A method (900) according to paragraph 59, wherein the predictive graph comprises indicia of a target zone (740) for the physiological characteristic.
Paragraph 64. A method (900) according to paragraph 63, further comprising predicting (908) whether the future measurements will leave the target zone (740) within a period of time.
Paragraph 65. A method (900) according to paragraph 64, further comprising: obtaining (910) an estimated time corresponding to when the future measurements will leave the target zone (740); and generating (912) an indicator of the estimated time for display with the predictive graph.
Paragraph 66. A method (900) according to paragraph 59, further comprising: generating a warning alarm when the future measurements indicate an alarm condition for the physiological characteristic of the monitored patient; and generating an alarm screen corresponding to the warning alarm.
Paragraph 67. A method (900) according to paragraph 59, further comprising extrapolating the empirical measurements to obtain extrapolated data, wherein the future measurements are based upon the extrapolated data.
Paragraph 68. A method (900) according to paragraph 59, further comprising the step of performing curve fitting on the empirical measurements to obtain curve fitted data, wherein the future measurements are based upon the curve fitted data.

## Claims

1. A monitor device (200) for a medical device system (100), the monitor device (200) comprising:
a display element (208);
a display controller/driver (418) coupled to the display element (208) and configured to generate a screen for rendering on the display element (208); and
a processing architecture (318) configured to:
analyze sensor data obtained by the monitor device (200), the sensor data indicating empirical measurements of a physiological characteristic of a monitored patient; and
estimate future measurements of the physiological characteristic based upon
the sensor data; wherein
the screen comprises a predictive graph (734) of the physiological characteristic that graphically indicates the empirical measurements and the future measurements.

2. A monitor device (200) according to claim 1, wherein the predictive graph (734) comprises indicia of a target zone (740) for the physiological characteristic.

3. A monitor device (200) according to claim 2, the processing architecture (318) being configured to predict whether the future measurements will leave the target zone (740) within a period of time.

4. A monitor device (200) according to claim 3, wherein:
the processing architecture (318) is configured to obtain an estimated time (746) corresponding to when the future measurements will leave the target zone (740); and
the screen comprises an indicator of the estimated time.

5. A monitor device (200) according to claim 4, wherein the indicator comprises a countdown timer.

6. A monitor device (200) according to claim 1, wherein:
the medical device system (100) is an insulin infusion system having a continuous glucose sensor transmitter (106); and
the sensor data comprises blood glucose data.

7. A monitor device (200) according to claim 1, the processing architecture (318) being configured to extrapolate the empirical measurements, resulting in extrapolated data, wherein the future measurements are based upon the extrapolated data.

8. A monitor device (200) according to claim 1, the processing architecture (318) being configured to perform curve fitting on the empirical measurements, resulting in curve fitted data, wherein the future measurements are based upon the curve fitted data.

9. A method (900) of operating a monitor device (200) for a medical device system (100), the method (900) comprising:
receiving (902) sensor data that indicates empirical measurements of a physiological characteristic of a monitored patient;
estimating (904) future measurements of the physiological characteristic based upon the sensor data;
generating (906) a predictive graph of the physiological characteristic, where the predictive graph graphically indicates the empirical measurements and the future measurements; and
displaying (918) the predictive graph at the monitor device.

10. A method (900) according to claim 9, wherein receiving (902) the sensor data comprises wirelessly receiving the sensor data from an infusion pump (104).

11. A method (900) according to claim 9, wherein receiving (902) the sensor data comprises wirelessly receiving the sensor data from a continuous glucose sensor transmitter (106).

12. A method (900) according to claim 9, wherein the predictive graph comprises indicia of a target zone (740) for the physiological characteristic, the method, further comprising:
predicting (908) whether the future measurements will leave the target zone (740) within a period of time;
obtaining (910) an estimated time corresponding to when the future measurements will leave the target zone (740); and
generating (912) an indicator of the estimated time for display with the predictive graph.

13. A method (900) according to claim 9, further comprising:
generating a warning alarm when the future measurements indicate an alarm condition for the physiological characteristic of the monitored patient; and
generating an alarm screen corresponding to the warning alarm.

14. A method (900) according to claim 9, further comprising extrapolating the empirical measurements to obtain extrapolated data, wherein the future measurements are based upon the extrapolated data.

15. A method (900) according to claim 9, further comprising the step of performing curve fitting on the empirical measurements to obtain curve fitted data, wherein the future measurements are based upon the curve fitted data.
